Europäisches Patentamt

(19) European Patent Office

Office européen des brevets

(11) Publication number : **0 569 777 A2**

## (12) EUROPEAN PATENT APPLICATION

(21) Application number : 93106884.5

(22) Date of filing : 28.04.93

(51) Int. Cl.$^5$ : **C12Q 1/37,** G01N 33/68, **C12N 9/64, A61K 37/64**

The applicant has subsequently filed a sequence listing and declared, that it includes no new matter.

(30) Priority : **11.05.92 US 880914**
**16.12.92 US 995660**

(43) Date of publication of application :
**18.11.93 Bulletin 93/46**

(84) Designated Contracting States :
**AT BE CH DE DK ES FR GB GR IE IT LI LU MC NL PT SE**

(71) Applicant : **MILES INC.(an Indiana corp.)**
**One Mellon Center 500 Grant Str.**
**Pittsburgh, PA 15219-2502 (US)**

(72) Inventor : **Tamburini, Paul P.**
**36 Misty Mountain Road**
**Kensington, CT 06037 (US)**
Inventor : **Dreyer, Robert N.**
**838 E. Center Street**
**Wallingford, Ct 06492 (US)**

(74) Representative : **Dänner, Klaus, Dr. et al**
**Bayer AG Konzernverwaltung RP Patente Konzern**
**D-51368 Leverkusen (DE)**

(54) Methods for detecting beta amyloid precursor protein processing enzymes.

(57)    Disclosed are methods for regulating formation of beta-amyloid protein with inhibitors of proteases specific for the Precursor to the Alzheimer's Disease beta-amyloid protein, such as inhibitors of aspartic protease, cathepsin D, and a chymotryptic-like serine protease ; a method for detecting in vitro protease activity for molecules which possess specificity necessary for the degradation of the Amyloid Precursor Protein ; and assays for identifying inhibitors of proteases specific for the Amyloid Precursor Protein.

EP 0 569 777 A2

Jouve, 18, rue Saint-Denis, 75001 PARIS

## FIELD OF THE INVENTION

The invention relates to methods for identifying proteolytic enzymes with specificity for processing the precursor to the Alzheimer's Disease beta-amyloid protein; methods for identifying inhibitors of proteases specific for the precursor to the beta-amyloid protein; and methods for regulating formation of beta-amyloid protein with inhibitors of proteases specific for the precursor to the beta-amyloid protein, such as inhibitors of aspartic protease, cathepsin D, and a chymotryptic-like serine protease.

## BACKGROUND

The present assays have utility in the identification of the proteases which control the rate of formation of amyloidic peptides in the brains of Alzheimer's Disease patients. As such, they can be used to isolate such proteases, and can also be used to identify protease inhibitors which can be used as therapeutics for Alzheimer's Disease. Described hereinbelow is the application of the assays to identify the aspartic protease, cathepsin D as a major amyloidogenic, protease for processing Amyloid Precursor Protein (hereinafter "APP"). Also provided is a partial characterization of a second, serine protease which can form amyloidic precursors from the APP holoprotein.

Alzheimer's Disease (hereinafter also abbreviated to "AD") is a progressive, degenerative disorder of the brain, characterized by progressive atrophy, usually in the frontal, parietal and occipital cortices. The clinical manifestations of AD include progressive memory impairments, loss of language and visuospatial skills, and behavioral deficits (McKhan et al., 1986, Neurology 34:939). Overall cognitive impairment is attributed to degeneration of neuronal cells located throughout the cerebral hemispheres (Price, 1986, Annu. Rev. Neurosci. 9:489).

Pathologically, the primary distinguishing features of the post-mortem brain of an AD patient are, 1) pathological lesions comprised of neuronal perikarya containing accumulations of neurofibrillary tangles; 2) cerebrovascular amyloid deposits; and 3) neuritic plaques. Both the cerebrovascular amyloid (Wong et al., 1985, PNAS 82:8729) and the neuritic plaques (Masters et al., 1985, PNAS 82:4249) contain a distinctive peptide simply designated, "A4" or "beta-amyloid".

Beta-amyloid is an insoluble, highly aggregating, small polypeptide of relative molecular mass 4,500, and is composed of 39 to 42 amino acids. Several lines of evidence support a role of beta-amyloid in the pathogenesis of AD lesions. For instance, beta-amyloid and related fragments have been shown to be toxic for PC-12 cell lines (Yankner et al., 1989, Science 245:417); toxic for primary cultures of neurons (Yankner et al., 1990, Science 250:279); and cause neuronal degeneration in rodent brains and corresponding amnestic response in the rodents (Flood et al., 1991, PNAS 88:3363; Kowall et al., 1991, PNAS 88:7247).

Kang et al., 1987, Nature 325:733, described the beta-amyloid protein as originating from and as a part of a larger precursor protein. To identify this precursor, a full-length complementary DNA clone coding for the protein was isolated and sequenced, using oligonucleotide probes designed from the known beta-amyloid sequence. The predicted precursor contained 695 residues and is currently designated, "APP 695" (Amyloid Precursor Protein 695).

Subsequent cloning of the gene encoding the APP proteins revealed that the A4 region was encoded on two adjacent exons (Lemaire et al., 1989, Nucleic Acids Res. 17:517), ruling out the possibility that A4 accumulation is the result of direct expression of an alternatively spliced mRNA. This implied that A4 accumulation must result from abnormal proteolytic degradation of the APP at sites both N- and C-terminal to the peptide region within the APP.

It has recently been demonstrated that inheritance patterns in rare forms of Familial Alzheimer's Disease show co-segregation with point mutations within the open reading frame of the APP gene, providing further evidence for a role of beta-amyloid in the pathogenesis of AD. These include mutations C-terminal to the beta-amyloid region (Goate et al., 1991, Nature 349:704; Yoshioka et al., 1991, Biochem Biophys Res Comm, 178:1141; Chartier-Harlin et al., 1991, Nature 353:844; Murrell et al., 1991, Science 254:97), as well as proximal to the N-terminus of the beta-amyloid region (Mullan et al., 1992, Nature Genetics 1:345). It has been speculated but not proven that such mutations might increase the rate of beta-amyloid peptide formation in Familial Alzheimer's Disease either by perturbing the normal sorting and or processing of APP; or by altering the recognition sites for specific proteases involved in the generation of beta-amyloid.

APP 695 is the most abundant form of APP found in the human brain, but three other forms exist, APP 714, APP 751 and APP 770 (Tanzi et al., 1988, Nature 351:528; Ponte et al., 1988, Nature 331:525; Kitaguchi et al., 1988, Nature 331:530). The different length isoforms arise from alternative splicing from a single APP gene located on human chromosome 21 (Goldgaber et al., 1987, Science 235:877; Tanzi et al., 1987, Science 235:880).

APP 751 and APP 770 contain a 56 amino acid Kunitz inhibitor domain, which shares 40% homology with Bovine Pancreatic Trypsin Inhibitor. Both these forms of APP have protease inhibitory activity (Kitaguchi et al., 1988, Nature 311:530; Smith et al., 1990, Science 248:1126), and at least one of these forms is probably what was previously identified as Protease Nexin II (Oltersdorf et al., 1989, Nature 341:144; Van Nostrand et al., 1989, Nature 341:546).

The physiological role for the amyloid precursor proteins has not yet been confirmed. It has been proposed to be a cell surface receptor (Kang et al., 1987, Nature 325:733); an adhesion molecule (Schubert et al., 1989, Neuron 3:689); a growth or trophic factor (Saitoh et al., 1989, Cell 58:615; Araki et al., 1991, Biochem Biophys Res Comm, 181:265; Milward et al., 1992, Neuron 9:129); a regulator of wound healing (Van Nostrand et al., 1990, Science 248:745; Smith et al., 1990, Science 248:1126); or play a role in the cytoskeletal system (Refolo et al., 1991, J. Neuroscience 11:3888).

Many studies have been performed to examine the role of altered APP expression in AD, but the results have been conflicting (for example, see review article: Unterbeck et al., 1990, Review of Biological Research in Aging, Wiley-Liss, Inc., 4:139).

Studies have also been performed to examine if changes in the relative amounts of the different forms of APP are responsible for amyloid accumulation. The results of such studies have been equally confusing, but have generally supported the conclusion that the relative expression levels of the Kunitz domain containing APP's are elevated in AD (Johnson et al., 1990, Science 248:854). Accordingly, transgenic animals expressing elevated APP 751 have been found to display cortical and hippocampal beta-amyloid reactive deposits (Quon et al., 1991, Nature 352:239).

Recent studies have shown that APP fragments extending from the N-terminus of A4 to the C-terminus of the full length APP molecule (referred to hereinafter as the "C-100 fragment", because it is comprised of approximately 100 amino acids) are also capable of aggregation both in vitro (Dyrks et al., 1988, EMBO J. 7:949), and in transfected cells (Wolf et al., 1990, EMBO J. 9:2079; Maruyama et al., 1990, Nature 347:566). Over-expression of the C-100 fragment in transfected P19 cells has been shown to cause cellular toxicity (Fuckuchi et al., 1992, Biochem Biophys Res Comm 182:165).

Furthermore, C-terminal fragments containing both the beta-amyloid and the C-terminal domains have been shown to exist in human brain (Estus et al., 1992, Science 255:726), and studies in transfected cell lines suggest that these fragments may be produced in the endosomal-lysozomal pathway (Golde et al., 1992, Science 255:728).

Collectively the above reports suggest that a single proteolytic cleavage of APP at the N-terminus of the A4 region is sufficient to initiate the pathophysiology associated with AD. Recent studies have shown that cultures of primary cells and cell lines (including APP transfectants) secrete 3 to 4 kDa peptides which posses the same N-terminus as beta-amyloid (1-42 amino acids), and could conceivably comprise full length beta-amyloid (Haas et al., 1992, Nature, 349:322; Shoji et al., 1992, Science, 258:126). Such peptides have also been found in the CSF of AD and non AD patients (Seubert at al.,1992, Nature, 359:325, Shoji et al 1992, Id.).

APP is also cleaved at a site within the A4 region in the physiological pathway for secretion of the APP extracellular domain (Esch et al., 1990, Science 248:1122; Wang et al., 1991, J. Biol. Chem. 266:16960). This pathway is operative in several cell lines and necessarily results in the destruction of the A4, amyloidic region of the precursor. Evidence that such a pathway is also operative in the human brain has been obtained (Palmert et al, 1989, Biochem. Biophys. Res. Comm. 165:182).

The enzymes responsible for the normal, non-pathological processing of APP have been termed "secretases". C-terminal fragments resulting from secretase action are smaller than the C-100 fragments (defined above) by 17 amino acids, and will hereinafter be referred to as the "physiological C-terminal fragment."

It has been postulated that the net pathological accumulation of A4 is controlled by the relative activities of the pathologic and physiologic pathways of APP degradation.

Thus, several possibilities exist to explain the accumulation of beta amyloid in the brains of persons afflicted with Alzheimer's Disease, as follows:

1) a deficiency in the activity or levels of the secretase(s) involved in the destruction of the amyloidogenic region;

2) altered cellular sorting of APP such that it might become exposed to proteases of the pathologic pathway;

3) an elevation in the levels of the pathologic preoteas(s);

4) a deficiency in the levels of degradative enzymes which otherwise degrade amyloid as fast as it is produced; or

5) an increased susceptibility of APP to pathologic proteolytic degradation caused by mutations in the APP amino acid sequence.

Relatively little is known about the regulation of APP sorting in the cell. A growing hypothesis is that altered phosphorylation at least in part due to altered protein Kinase C activity causes altered APP trafficking, ultimately leading to changes in APP processing (Buxbaum et al., 1990, Proc Natl Acad Sci USA 87:6003). Thus, treatments designed to alter cellular phosphorylation have caused both qualitative and quantitative changes in the pattern of APP C-terminal fragments.

More Recently, (Nitsch et al., 1992, Science 258:304) it was shown that transfection of cell lines with certain acetylcholine receptor types followed by receptor activation caused an increase in APP processing and secretion, in a process concluded to arise by changes in protein kinase activity. Beside implicating a role for altered phosphorylation, this latter study provides a link between plaque pathology and the established perturbations in cholinergic nerve function charateristic of Alzheimer's Disease.

Despite the above observations, there is currently insufficient knowlege of APP sorting to enable the design of a selective and specific therapeutic agent that could restore balance to any underlying alterations of cellular sorting.

Beta-amyloid must be formed by the direct action of a protease(es). The identification of the so called 'pathologic' brain proteases responsible for the C-100 or beta-amyloid formation is an essential step in an effort to develop therapeutic protease inhibitors designed to block amyloid accumulation. Identification of such enzymes requires the development of specific assays for the activity of such proteases which would allow one to specifically measure the activity of the proteases in the presence of other brain proteolytic enzymes which are present in brain extracts.

Such assays are then used to detect the protease during protease purification. Finally, the assays can be used to measure the effect of potential inhibitors of the enzyme such as is required in pharmaceutical screening for lead therapeutic compounds.

Several studies have undertaken the purification and characterization of both the secretases and purported pathologic proteases. Initial studies utilized assays featuring synthetic peptide substrates that only mimicked the expected cleavage sites within APP. While such assays are useful for measuring the in vitro activity of a purified protease, they rarely possess sufficient specificity to allow detection of one protease in a mixture of proteases such as would be required to monitor a protease purification. Thus, these peptidase assays failed to provide the necessary protease specificity, and the peptidase activities thus quantified were used without success to pursue the purification of candidate APP processing enzyme activities from human brain tissue. Prior to the present disclosure, no credible candidate protease(s) for either process have emerged, and the results of the various studies have been conflicting.

For example, the numerous available studies have proposed that the pathologic protease is: lysosomal in origin (Cataldo et al., 1990, Proc. Natl. Acad. Sci USA 87:3861; Haas et al., 1992, Nature 357:500); a calcium dependent serine protease or a metal dependent cysteine protease (Abrahams et al., 1991, An. N.Y. Acad. Sci. 640:161); Calpain I (Siman et al., 1990, J. Neuroscience 10:2400); a multicatalytic protease (Ishiura et al., 1989, FEBS. Lett. 257:388); a serine protease (Nelson et al., 1990, J. Biol Chem. 265:3836); thrombin (Igarashi et al., 1992, Biochem Biophys Res. Comm. 185:1000); or a zinc metallo-peptidase (WIPO application, WO92/07068 by Athena Neurosciences, Inc.).

Similar inconsistencies have arisen in the efforts to identify the secretase, which has been claimed to be: a metallo-peptidase (McDermott et al., 1991, Biochem. Biophys. Res. Comm. 179:1148); an acetylcholinesterase associated protease (Small et al., 1991, Biochemistry 30:10795); Cathepsin B (Tagawa et al., Biochem. Biophys. Res. Comm. 177:377); or a plasma membrane associated protease of broad sub-site specificity (Sisodia, 1992, Proc Nat'l Acad Sci USA 89:6075; Maruyama et al., 1991, Biochem Biophys Res Comm. 179:1670).

The general lack of success of past and current efforts to identify the nature of the APP processing enzymes have stemmed from poor specificity of the assays employed, and from the complex heterogeneity of proteases associated with the cerebral tissue.

The present disclosure describes a method which identifies some of the APP processing enzymes with specific assays based on the proteolytic degradation of recombinant APP in combination with immunochemical detection of the reaction products. The assays of the present invention identify human brain proteases that possess the correct specificity and appropriate localization to play a role in the formation of beta-amyloid from the APP.

The format of the presently disclosed assays in conjunction with the identified proteases afford the capacity to process reasonably large numbers of samples and yields good sensitivity due to the immunochemical method of detection. Furthermore, the simplicity of the assay allows for ready adaptation for routine use by lab technicians and yields consistent, reproducible results. These and other improvements are described hereinbelow.

## SUMMARY OF INVENTION

One goal of the presently disclosed invention is to provide a method for discovering drugs that can be used to treat Alzheimer's Disease patients. As stated previously, the proteolytic degradation of APP to yield the 39 to 42 amino acid peptide beta-amyloid is the first step in the pathophysiological process of amyloid placque formation. Several lines of evidence point to a causative role of beta-amyloid and the amyloid placques in the neuro-degeneration characteristics found in the AD brain. These include:

i) the co-localization of plaque material with degenerating neurons and dystrophic neurites (reviewed in Price et al., 1989, BioEssays 10:69;

ii) evidence that beta-amyloid can be toxic to neurons in culture (Yankner et al., 1990, Science 250:270);

iii) evidence that beta-amyloid is associated with neuronal degeneration and altered memory when tested in certain animal models (Flood et al., 1991, PNAS 88:3363; Kowall et al., 1991, PNAS 88:7247); and

iv) co-segregation of certain forms of inherited AD with point mutations in the amyloid precursor protein (Goate et al., 1991, Nature 349:704; Yoshioka et al., 1991, Biochem Biophys Res Comm 178:1141; Chartier-Harlin et al., 1991, Nature 353:844; Murrell et al., 1991, Science 254:97; Mullan et al., 1992, Nature Genetics 1:345).

Thus, proteolytic conversion of APP to beta-amyloid appears to be an essential step in the pathogenesis of AD and, as such, an important target for therapeutic intervention. Identification of the relevant protease activities, as well as the development of suitable in vitro screening assays, are therefore essential prerequisites for the development of therapeutic protease inhibitors that could be used as treatments to block amyloid placque formation in Alzheimer's Disease patients.

The present invention contains two developments which can be used to discover inhibitors of proteolytic beta-amyloid formation:

1) An in vitro assay comprising a holo-APP substrate and either a highly purified protease that degrades APP or a crude biological extracts containing unidentified proteases that can degrade APP.

The assay enables the detection of in vitro APP degradation activity to yield C-terminal APP fragments. When used with crude biological extracts, the assay can be used to monitor the purification of, or to characterize the protease responsible for the detected activity.

Additionally, when used with either a purified protease or a crude biological extract containing un-identified APP degrading enzyme activities, the assay can be used to measure the inhibition of the APP processing activity by chemical or biological compounds that are co-incubated in the assay mixture. Inhibitory compounds thereby identified can have application as therapeutic inhibitors of the in vivo amyloid placque formation characteristic of Alzheimer's Disease patients.

2) The identification and purification of specific proteases from human brain that can form amyloidic or pre-amyloidic APP C-terminal fragments when used in conjunction with the in vitro assay system described in (1), above.

Such enzymes include the aspartic protease, cathepsin D and a chymotryptic-like serine protease distinct from cathepsin G and inhibited by TPCK and alpha-2 antiplasmin and chymotrypsin inhibitor II from potato. The identification of cathepsin D is particularly significant. We show that cathepsin D is able to form C-100 like and beta-amyloid like fragments of 10.0 kDa and 5.6 kDA size, respectively.

This discovery enables the use of any purified or isolated cathepsin D to perform a search for inhibitors of its activity using either the in vitro assay described in (1), above or simpler high throughput peptidase assays such as those described in the present invention.

Furthermore, since much is known about the specificity of cathepsin D as well as the design of specific aspartic protease inhibitors, identification of cathepsin D as an amyloidogenic protease enables both the development of specific cathepsin D inhibitors using established methods, as well as the utilization of established cathepsin D inhibitors.

Also shown below is that cathepsin D, unexpectedly, hydrolyzes APP at the peptide bond between Glu-Val (preferred specificity of cathepsin D is, ordinarily, between hydrophobic residues). This information can be used further in the design of cathepsin D inhibitors.

As mentioned above, inhibitory compounds thereby identified have application as therapeutic inhibitors of the in vivo amyloid placque formation characteristic of Alzheimer's Disease patients.

APP degrading enzymes identified by use of the present invention can be purified and used to:

i) develop immunochemical reagents necessary to further correlate the co-localization of protease with AD brain pathology; and

ii) isolate the corresponding protease cDNA. The cloned cDNA can then be used to construct transgenic animal models for AD in which the effect of protease overexpression can be assessed.

Assays incorporating synthetic peptide substrates are useful for in vitro enzymological studies of highly purified protease preparations, but are generally of insufficient specificity to enable the selective detection of a desired protease activity in crude biologic extracts containing a plethora of proteases. For instance, brain tissue is abundant with a wide and varied range of peptide processing and degrading enzymes, which may explain why efforts to isolate specific brain APP degrading proteases with synthetic peptide substrates have been unsuccessful (see Background section, above).

Accordingly, in Example 3, the present disclosure shows that synthetic peptide assays lead to the identification of several peptidases which are unable to degrade APP to yield C-terminal fragments under the specified assay conditions, and that the pattern of APP degrading proteases does not resemble in any way the corresponding pattern of brain peptidases.

A more definitive approach to this problem is the utilization of holo-APP as a substrate, in conjunction with a method of assessing its specific degradation following incubation with protease containing fractions. To this end, the present disclosure describes such a method, wherein the enzymic degradation of recombinant APP by brain protease fractions is monitored by immunoblot using antibodies to the C-terminal region of APP.

Our assay procedure focuses on the formation of C-terminal fragments from APP of size sufficient to include the full length beta-amyloid peptide (a process requiring endoproteolysis, N-terminal to the A4 region).

Human brain tissue (non-AD control or Alzheimer's) is homogenized and then sub-fractionated into a soluble fraction (hereinafter "S"), a post 15,000 g pellet (hereinafter "P-2"), and a microsomal fraction (hereinafter "M"), using conventional ultra-centrifugation. The membranous M and P-2 fractions are solubilized with a Triton X-100 preparation. The resulting soluble fractions from M and P-2, as well as the S fraction, are then separately subjected to chromatography on a Mono-Q strong anion exchange column which results in separation of different brain proteases.

Using a synthetic peptide that mimicks the amino acid sequence surrounding the N-terminus of beta-amyloid, the peptidase activity of individual mono-Q fractions from the purification of M, soluble and P-2 fractions is assessed. Contigous pools of column fractions are made based on the recovery of discrete peaks of peptidase activity.

The pools of peptidase activity are used to establish assay conditions for the detection of proteolytic degradation of highly pure recombinant amyloid precursor protein purified from a transfected CHO cell line. An immunoblot assay is developed in which antibodies directed either to the APP c-terminal domain or the beta-amyloid region are used to locate c-terminal APP fragments. The assay is used to identify six potentially different proteolytic activities capable of forming APP c-terminal fragments of a sizes large enough to potentially contain full length beta-amyloid. The recovery of APP degrading activity amongst the mono-Q pools is not found to correlate well with the peptidase activity profiles established in step 2. Inhibitor studies reveal that the APP degrading activities include both serine and aspartic protease activities.

The use of the peptidase assay for monitoring enzyme purification is abandoned. Larger supplies of recombinant APP are obtained by expression in a baculovirus directed insect cell system, enabling use of the APP degradation assay as the primary method to monitor APP degrading activity during protein purification. A major aspartic protease activity is identified in fractions from the mono-Q purification of the P-2 fraction.

Further purification and characterisation experiments demonstrate that the enzyme is cathespin D. The cathepsin D is shown to hydrolyse holo-APP forming a beta-amyloid like fragment of 5.6 kDa.

Aprotinin sepaharose affinity chromatography is used to attempt to isolate aprotinin sensitive APP degrading activities identified above. A chymotrypsin like serine protease activity is partially purified that can degrade APP to form specific C-terminal fragments of 11, 14 and 18 kDa, that are shown by immunochemical means to contain full length beta-amyloid.

Through this procedure, we have identified several brain protease activities that play, with high probability, a role in amyloidogenic degradation of APP. Each of the identified or unidentified activities described herein can in conjunction with the APP degradation assay be used to screen for selective protease inhibitors of therapeutic value.

As used herein, "APP substrate" shall mean full length APP, whether derived by isolation or purification from a biological source or by expression of a cloned gene encoding APP or its analogs, and fragments of any such protein, including fragments obtained by digestion of the protein or a portion thereof, fragments obtained by expression of a gene coding for a portion of the APP protein, and synthetic peptides having amino acid sequences corresponding to a portion of the APP protein.

APP substrates for the assays of the present invention can be provided as a test reagent in a variety of forms. Although preferably derived from, or corresponding at least in part with the amino acid sequence of, APP 695, derivatives or analogs of other APP isoforms (supra) are contemplated for use in the present method as well. APP 695 can be obtained by biochemical isolation or purification from natural sources such as described in Schubert et al., 1989, Proc. Natl. Acad. Sci. 86:2066; or by expression of recombinant DNA clones encoding the protein or a functional portion thereof (Knops et al., 1991, J. Bio. Chem. 266:7285; Bhasin et al.,

1991, Proc. Natl. Acad. Sci. 88:10307).

The fragments of the APP protein will comprise a sequence of amino acids sufficient for recognition and cleavage by the pertinent proteolytic test sample activity (supra). Isolation of APP from biological material usually will involve purification by conventional techniques such as chromatography, particularly affinity chromatography. Purified APP or fragments thereof can be used to prepare monoclonal or polyclonal antibodies which can then be used in affinity purification according to conventional procedures. Resulting purified APP material can be further processed, e.g., fragmented, by chemical or enzymatic digestion. Useful fragments will be identified by screening for desired susceptibility to the pertinent proteolytic test sample activity (supra).

As previously stated, the APP substrate can also be prepared by expression of recombinant DNA clones coding for APP or a portion thereof. The cloned APP gene may itself be natural or synthetic, with the natural gene obtainable for cDNA or genomic libraries using degenerate probes based on known amino acid sequences (Kang et al., 1987, Nature 325:733). Other techniques for obtaining suitable recombinant DNA clones, as well as methods for expressing the cloned gene, will be evident to the worker in the field.

A variety of convenient methods are applicable to the detection of proteolytic cleavage of the APP substrate in the presence of the test sample. Several of the presently more preferred methods are described below, however, it will be recognized by the skilled worker in the field that many other methods can be applied to this step without departing from the inventive features hereof. In general, any method can be used for this purpose which is capable of detecting the occurrence of proteolytic cleavage of the APP substrate. Such can be afforded by appropriate design of the APP substrate such that cleavage produces a signal producing species, e.g., an optically responsive product such as a colored or fluorescent dye.

Another principal approach involves the sensitive detection of one or more cleavage products such as by immunoassay. Presently, such cleavage product is preferentially a C-terminal fragment of the APP substrate; however, any fragment which appears upon incubation with samples can be the object of detection.

The detection of one or more cleavage products characteristic of the pathologic proteolytic activity can be accomplished in many ways. One such method, which is further exemplified in the examples which follow, involves the procedure commonly known as Western blot. Typically, after the incubation of APP with test sample, gel electrophoresis is performed to separate the components resulting in the reaction mixture. The separated protein components are then transferred to a solid matrix such as a nitrocellulose membrane.

An antibody specific to a fragment characteristic of APP degradation is then reacted with the components fixed to the membrane and detected by addition of a secondary enzyme-labeled antibody conjugate. The location of the resulting bound conjugate is developed with a chromogenic substrate for the enzyme label.

A variety of immunoassay formats which are amenable to currently available test systems can also be applied to the detection of APP fragments. Typically, the APP substrate will be incubated with the test sample and resulting intact APP rendered immobilized (such as by capture onto a solid phase), or alternatively, the test sample is incubated with an immobilized form of the APP substrate. Proteolytic cleavage is then detected by reacting the immobilized APP substrate with an antibody reagent directed to a portion of the APP substrate which is cleaved from the APP substrate or which defines the cleavage site.

The antibody reagent can comprise whole antibody or an antibody fragment comprising an antigen combining site such as Fab or Fab', and can be of the monoclonal or polyclonal type. The detection of antibody reagent bound to the immobilized phase is indicative of the absence of the characteristic proteolytic cleavage. Conversely, loss of antibody binding to the immobilized phase is indicative of APP cleavage. The detection of binding of the antibody reagent will generally involve use of a labeled form of such antibody reagent or use of a second, or anti-(antibody), antibody which is labeled.

Capture or immobilization of APP can be accomplished in many ways. An antibody can be generated specific to an epitope of APP which is not on the cleavable fragment. Such an antibody can be immobilized and used to capture or immobilize intact APP. Alternatively, a ligand or hapten can be covalently attached to APP and a corresponding immobilized receptor or antibody can be used to capture or immobilize APP. A typical ligand:receptor pair useful for this purpose is biotin:avidin. Examples of haptens useful for this purpose are fluorescein and digitoxigenin.

The solid phase on which the APP substrate is immobilized or captured can be composed of a variety of materials including microtiter plate wells, test tubes, strips, beads, particles, and the like. A particularly useful solid phase is magnetic or paramagnetic particles. Such particles can be derivatized to contain chemically active groups that can be coupled to a variety of compounds by simple chemical reactions. The particles can be cleared from suspension by bringing a magnet close to a vessel containing the particles. Thus, the particles can be washed repeatedly without cumbersome centrifugation or filtration, providing the basis for fully automating the assay procedure.

Labels for the primary or secondary antibody reagent can be selected from those well known in the art. Some such labels are fluorescent or chemiluminescent labels, radioisotopes, and, more preferably, enzymes for this purpose are alkaline phosphatase, peroxidase, and β-galactosidase. These enzymes are stable under a variety of conditions, have a high catalytic turnover rate, and can be detected using simple chromogenic substrates.

Proteolytic cleavage of the APP substrate can also be detected by chromatographic techniques which will separate and then detect the APP fragments. High pressure liquid chromatography (HPLC) is particularly useful in this regard. In applying this technique, a fluorescently tagged APP substrate is prepared. After incubation with the test sample, the reaction mixture is applied to the chromatographic column and the differential rate of migration of fluorescent fragments versus intact APP is observed.

## BRIEF DESCRIPTION OF THE DRAWINGS

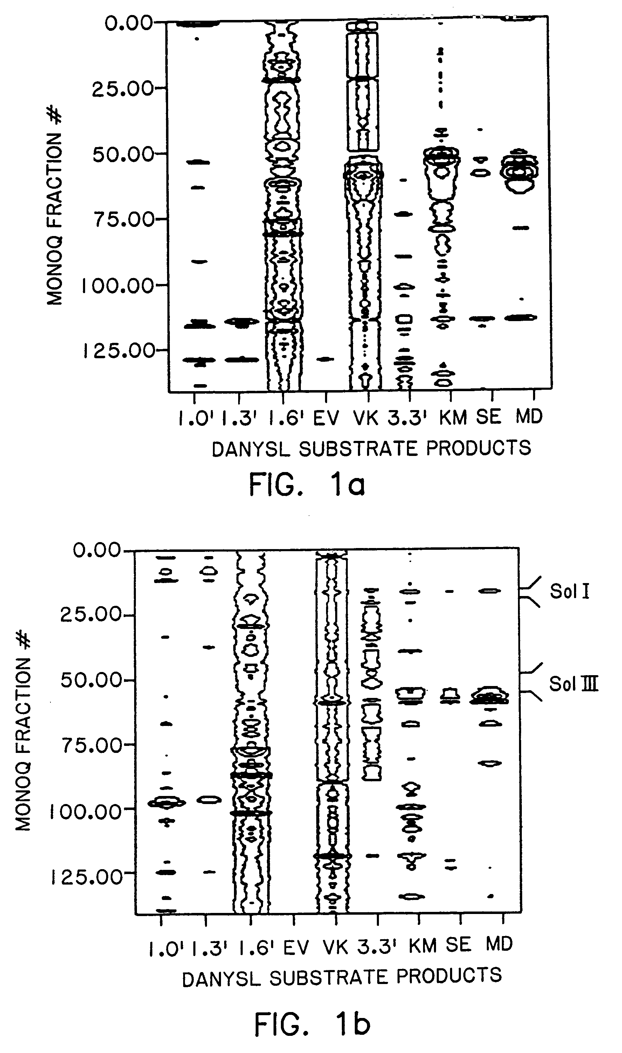

**Figures 1a-1f show two dimensional contour plots of peptidase activities of control compared to AD human cortex subfractions.**

Subfractions were prepared according to Example 1, by ion-exchange (mono-Q) separation of P-2, S and M fractions. Enzymatic cleavage of N-dansyl-Ile-Ser-Glu-Val-Lys-Met-Asp-Ala-Glu-Phe-Arg-His-Asp-Asp-Asp-Asp (SEQ ID NO: 1) by Mono-Q fractions was performed as described in Example 3. Each plot shows the relative amounts of each flourescent product (abscissa) obtained by incubation of each mono-Q fraction (ordinate) under the same incubation conditions. The amount of product is represented vertically by contour lines. Greater numbers of contour lines indicate greater amounts of a particular product. Mono-Q fractions from control S (a), AD S (b), control M (c), AD M (d), control P-2 (e), AD P-2 (f), were subjected to analysis. The roman numerals on the right hand ordinate of the three AD plots locate pooled regions described in Example 3, and which were then assayed according to Example 8, and found to contain significant APP degrading activity.

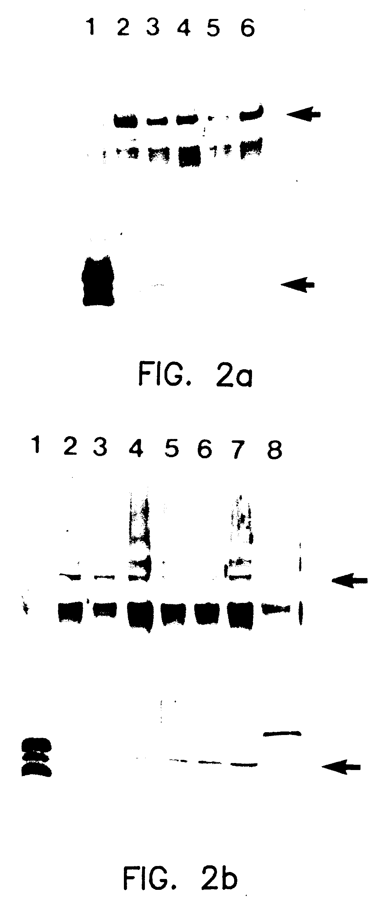

**Figures 2a-2f depict immunoblot analysis of the APP 695 degrading activity associated with selected Mono-Q pools from the ion-exchange separation of M, S or P-2 fractions derived from AD cortex.**

The pools were made based on their content of peptidase activity as described in Example 3. Immunoblot assays were performed as described in Example 8. Representative assays for the following pools are shown:

**Figure 2a: Activity associated with P-2 pool V:**

APP was present in each of lanes 2 to 6. C-100 from PMTI 73 (lane 1), no P2-V blank (lane 2), P2-V (lane 3), P2-V plus EDTA (lane 4), P2-V plus methanol (lane 5), P2-V plus pepstatin A in methanol (lane 6).

**Figure 2b: Activity associated with M pool III:**

APP was present in lanes 2 to 7.
C-100 from PMTI 73 (lane 1), M-III plus cystatin C (lane 2), M-III plus aprotinin (lane 3), M-III plus captopril (lane 4), M-III plus EGTA (lane 5), M-III plus N-dansyl-Ile-Ser-Glu-Val-Lys-Met-Asp-Ala-Glu-Phe-Arg-His-Asp-Asp-Asp-Asp (SEQ ID NO: 1) (lane 6), M-III without inhibitor (lane 7), prestained molecular weight markers (lane 8).

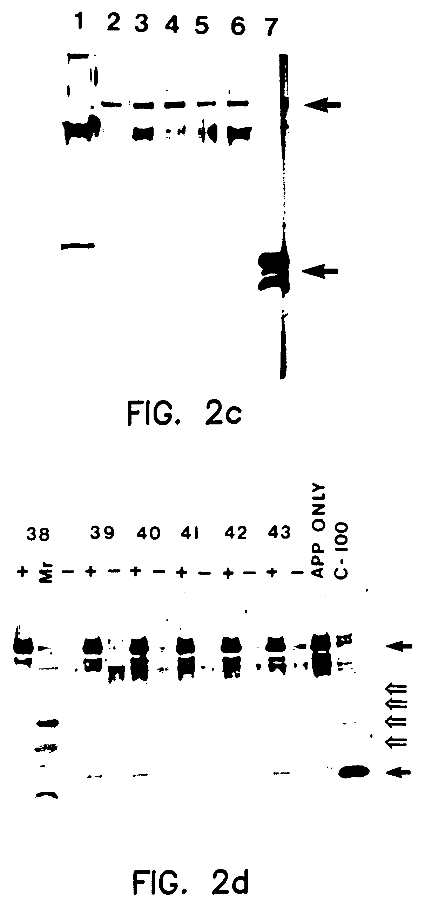

**Figure 2c: Activity associated with S pool I:**

APP was present in each of lanes 2 through 6. S-I plus N-dansyl-Ile-Ser-Glu-Val-Lys-Met-Asp-Ala-Glu-Phe-Arg-His-Asp-Asp-Asp-Asp (SEQ ID NO: 1) (lane 2), S-I plus EGTA (lane 3), S-I plus captopril (lane 4), S-I plus aprotinin (lane 5), S-I plus cystatin C (lane 6), C-100 from PMTI 73 (lane 7). Lane 1 contains prestained molecular weight markers.

**Figure 2d: Activity recovered in individual mono-Q fractions from the separation of AD P-2:**

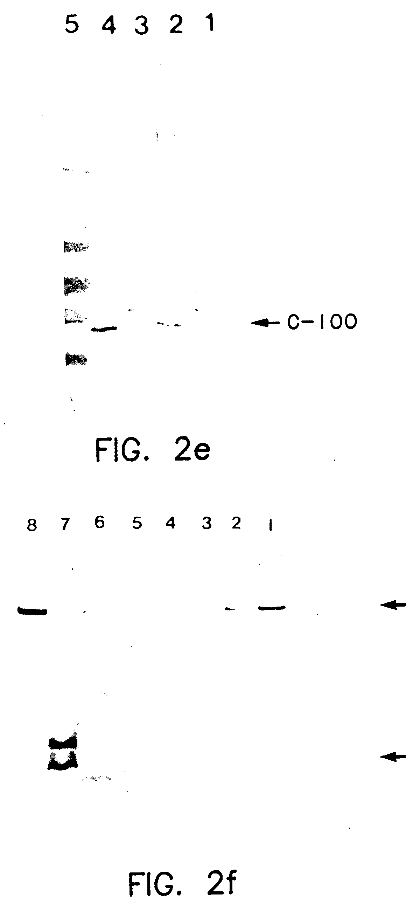

Mono-Q fractions 38 to 43 corresponding to the conductance region in which P-2 pool VII is otherwise observed were individually examined for APP degrading activity. For each fraction, the incubation was carried out both in the absence (-) or presence (+) of recombinant APP 695. The fraction numbers are located on Figure 2. The C-100 standard used was from PMTI 100. Mr indicates molecular size markers.

**Figure 2e: Comparison of the position of migration of C-100 products directed either by PMTI 73 or PMTI 100:**

The protein product of PMTI 73 (lanes 1 and 3) and PMTI 100 (lanes 2 and 4) are shown in comparison with molecular markers (lane 5).

**Figure 2f: Typical time course of product formation:**

APP plus M-III were analyzed at time t= 0 h (lane 1), 5 h (lane 2), 20.5 h (lane 3), 44.5 h (lane 4), and 55 h (lane 5). Molecular weight markers (lane 6), C-100 PMTI 73 (lane 7), and APP without M-III (lane 8), are also shown.

For each of Figures 2a through 2f, above, migration was from top to bottom. In 2a-2d and 2f, the upper solid arrow locates the position of migration of holo-APP, and the lower solid arrow locates the position of migration of C-100. In Figure 2d, the open arrows locate the positions of migration of putative oligomers of the enzymically generated C-100 fragment. The concentrations of all inhibitors are listed in Table 4, below.

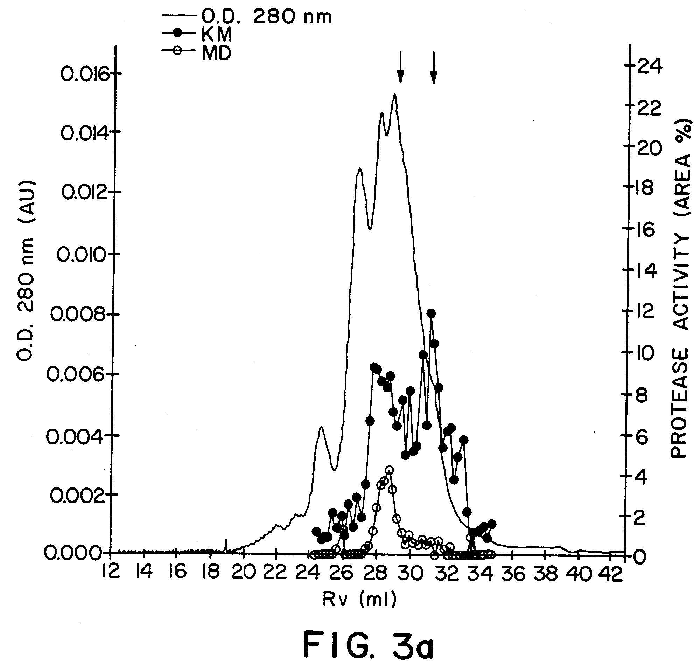

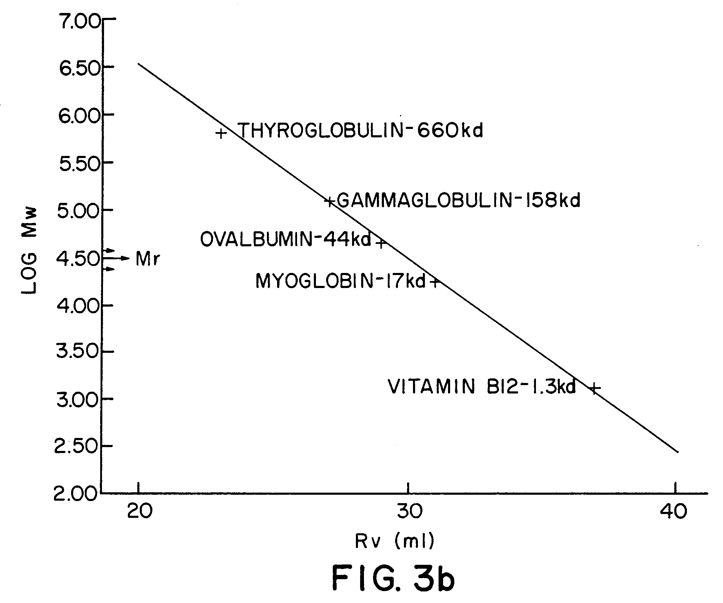

**Figures 3a and 3b depict results from further purification of P-2 pool VII by gel filtration.**

**Figure 3a:** P2-pool VII fractions from Mono-Q 10/10 chromatography were pooled, concentrated to 0.25 ml and applied to a tandem arrangement of two Superose 6HR 10/30 columns equilibrated in 10mM tris HCl buffer pH 7.5 containing 150mM NaCl. Elution was performed at a flow rate of 0.3 ml/min, and column eluent was monitored at 280 nm. Fractions (0.24 ml) were collected and subjected to both peptidase activity, and APP degradation assay using the immunoblot. The arrows locate the region of the chromatogram in which the APP degrading activity was recovered. Also shown are the peptidase activities associated with both K-M (closed circles) and M-D (open circles) bond cleavage. Chromatography was performed at 22°C.

**Figure 3b:** The migration of the APP degrading activity relative to the indicated standard proteins of known molecular weight was used to calculate an Mr apparent of the APP degrading protease which is listed in Example 8.

**Figure 4 shows Peptide Epitope Mapping of Murine Monoclonal Antibody C286.8A Raised Against the Beta-amyloid Peptide.**

Micro-titre plates were coated with 50 ng of synthetic APP 695 (597-638) (beta-amyloid 1-42), blocked, then incubated with 100 ul of C286.8A (80 ng of IgG) which had been preincubated (60 min at room temperature) in the presence or absence of the indicated concentrations of competitor peptide. Note, that the peptide 1-7 refers to peptide SEQ ID NO: 1. Following incubation for 60 min at room temperature, plates were washed, and the amount of bound antibody determined by development with horseradish peroxidase-coupled goat-anti mouse polyclonal antibody according to standard procedures (Wunderlich et al., 1992, J. of Immunol. Methods 147:1). Percent competition (% C) of antibody binding to the plate was calculated from the absorbance at 450 nm data using the following equation:

$$\% \; C \; = \; 1.0 \; - \; \frac{O.D. \; (+ \; competitor) \; - \; O.D. \; blank}{O.D. \; (- \; competitor) \; - \; O.D. \; blank} \times 100$$

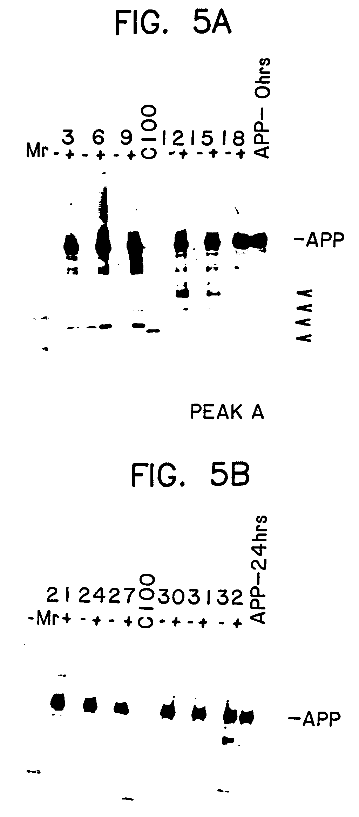

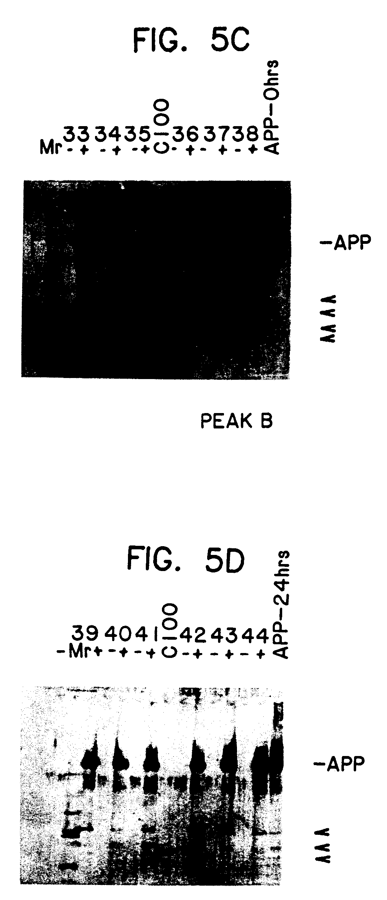

**Figure 5: APP695 processing activity recovered in ion-exchange fractions from the purification of human brain P-2 subfraction.**

A total of 123 fractions were collected from the column. The first 32 fractions corresponded to the load and wash phase. The salt gradient started at fraction 33. Screens of fractions 3 to 18 (panel a), 21 to 32 (panel b), 33 to 38 (panel c), and 39 through 44 (panel D), are shown. For each fraction, the incubation was performed in both the absence (-) and presence (+) of APP695 substrate. Incubations of APP695 for zero or 24 hr is located where appropriate. Incubations were performed as follows. Baculo-derived holo-APP695 (80 nM) was incubated with 5ul of each column fraction in a total of 15 ul containing 100 mM Mes buffer pH 6.5, 0.008 % (v/v) triton X-100, 160 mM NaCl, 6.7 mM tris (from the APP stock). Reactions were terminated after 24 h by addition of SDS-PAGE sample buffer. Immunoblots were developed using the C-terminal polyclonal antiserum of Example 6, as described in Example 8. The arrows locate the product fragments. Fractions 45 to 86 were also tested but showed relatively little activity (therefore not shown). Peak A and B locate the major activities.

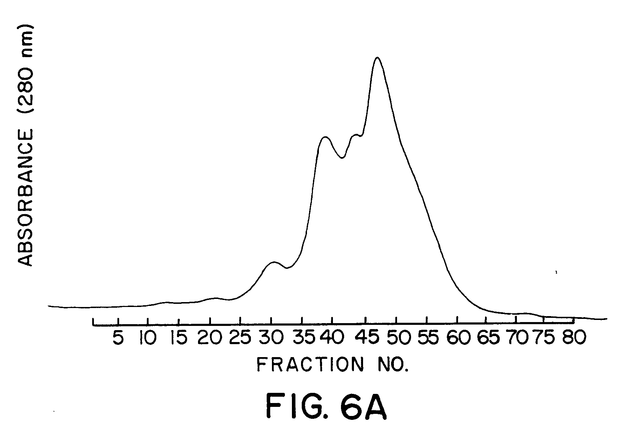

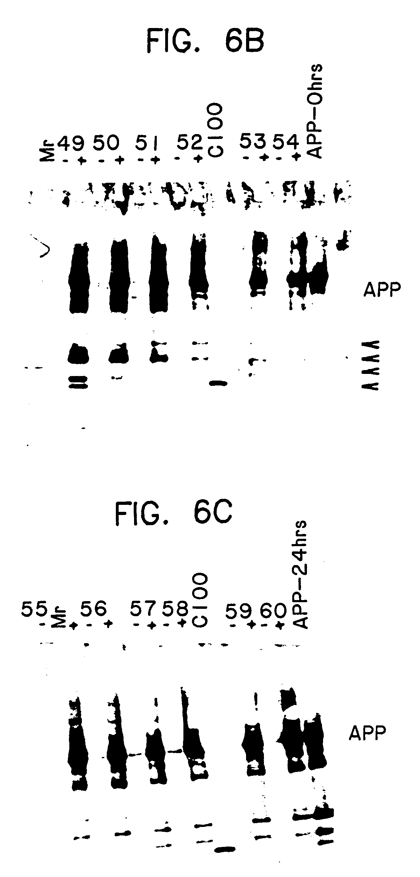

**Figure 6 shows results of purification of P-2 derived APP degrading activity on gel filtration: correlation with the elution of cathepsin D.**

Panel (a) A 280 nm elution profile for the purification of P-2 peak B on a superose 6HR column. Panels (b) and (c) corresponding APP c-terminal processing activity in the eluted fractions 49 to 60, determined essentially as described in Example 5. Arrows locate major product bands. Panels (d) and (e), Immunoblot analysis of eluted fractions using a rabbit polyclonal antibody to cathepsin D (1/300, dilution). The arrows locate the position of migration of immunoreactive bands. Human liver cathepsin D was also analyzed for comparison.

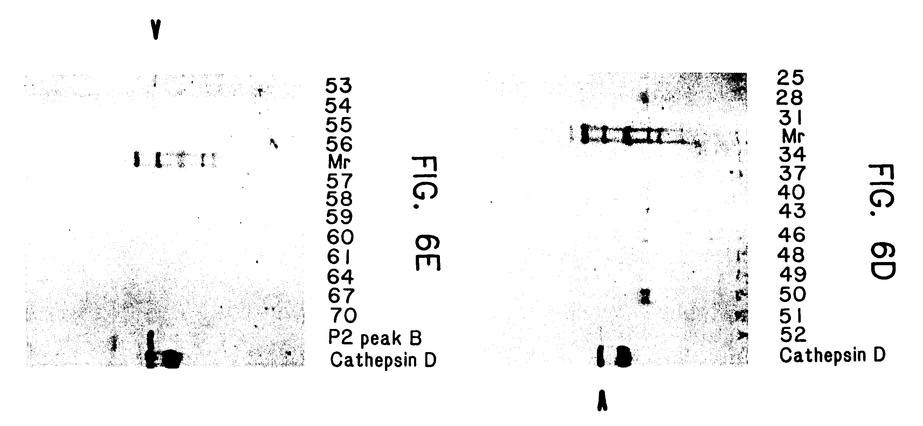

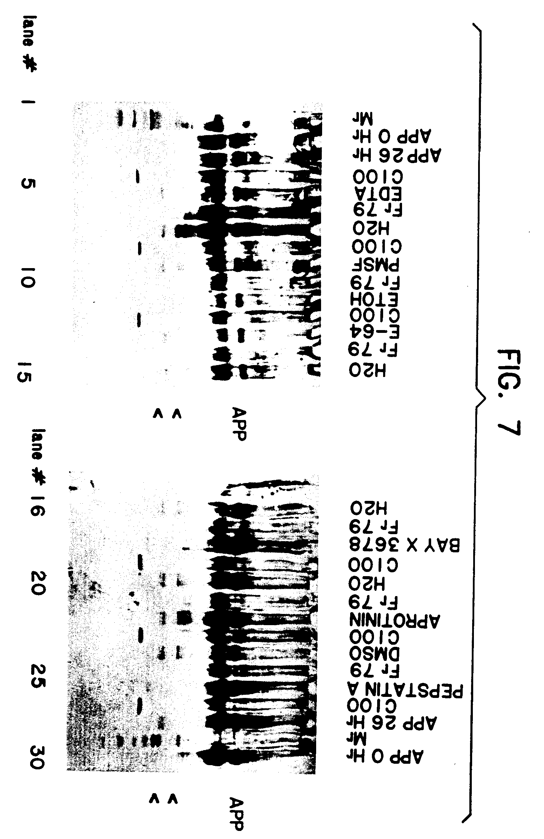

**Figure 7 depicts protease inhibitor specificity of protease activities isolated from the P-2 subfraction.**

Reactions (32 ul) were initiated at 37°C by APP addition to achieve the following initial component conditions: P-2 enzyme (2.54 ug/ml) fraction from the 15-25 kDa region of gel filtration (Figure 6); APP (168 nM), in 96 mM Mes buffer pH 6.5. Reactions were terminated after 26 hr by addition of 15 ul of 3X sample buffer, and subject to immunoblot analysis using a 1/1000 dilution of the rabbit polyclonal antiserum to the APP C-terminus. The effect of addition of the following inhibitors is shown: No inhibitor (lanes 7 and 20), 1 mM EDTA (lane 5); 400 uM PMSF in ethanol (lane 9); ethanol alone (lane 11); 100 uM E-64 (lane 13); 10 ug/ml aprotinin (lane 22); 100 uM pepstatin A in DMSO (lane 26); DMSO only (lane 24). The effect of incubation of APP for 0hr (lanes 2 and 30) and 26 hr (lanes 3 and 28) are also shown. Lanes 6, 10, 14, 17, 21 and 25 contained enzyme alone. Lanes 4, 8, 12, 19, 23, and 17 contained the C-100 standard. Prestained molecular weight markers are present in lanes 1 and 29. The 18 and 28 kDa C-terminal product fragments are located with arrows.

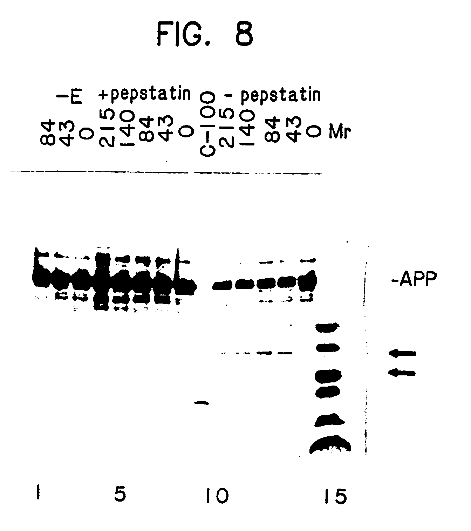

**Figure 8 shows time course of Cathepsin D catalysed APP cleavage monitored using an antibody to the APP c-terminal domain.**

Panel (a) shows time course of APP proteolysis by cathepsin D in the absence (lanes 10-14) or presence (lanes 4-8) of 86 uM pepstatin A. APP was also incubated alone (lanes 1-3). The numbers indicate the time (hr) after initiation of reactions. Reactions were initiated at 37°C by the addition of APP to achieve the following initial component concentrations: APP (82 nM), cathepsin D (9.2 ug/ml),in 89 Mes buffer pH 6.5. Samples without pepstatin received the same amount of solvent (1.3 % v/v methanol). At t=0, 43, 84, 140 and 215 minutes aliquots (15 ul) were removed mixed with 7.5 ul of SDS-PAGE sample buffer and subject to immunoblot analysis using the Rabbit antiserum to the APP C-terminal domain. The 18 and 28 kDa reaction products are located with arrows.

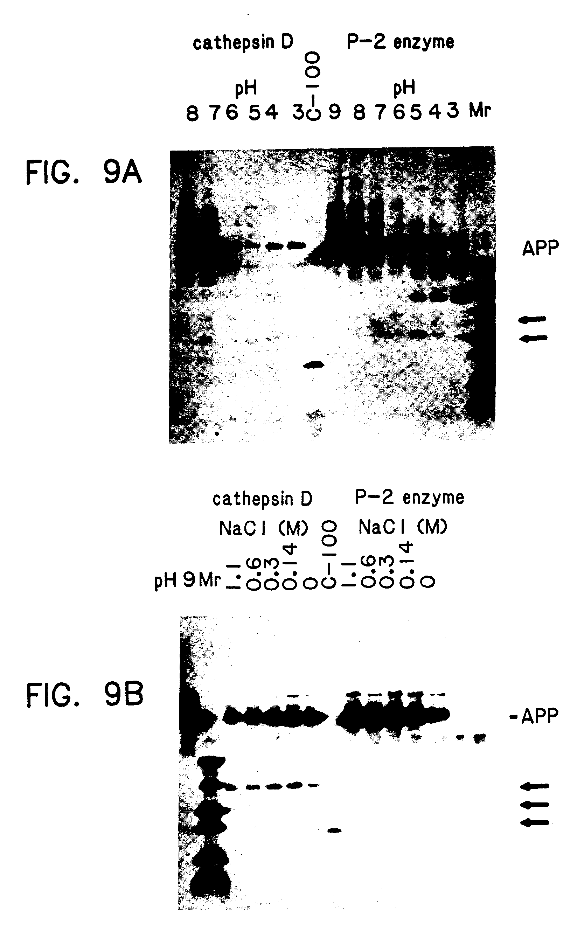

**Figure 9 depicts pH and ionic strength dependence of APP C-terminal processing by the P-2 derived enzyme or cathepsin D.**

Panel (a) shows pH dependence observed with cathepsin D and the P-2 enzyme (peak B, Figure 5 following gel filtration chromatography, Figure 6). Panel (b) is ionic strength dependence for both enzymes at pH 6.5. Reactions were initiated at 37°C by enzyme addition to achieve the following initial component concentrations: cathepsin D (9.2 ug/ml) or P-2 enzyme from figure 6 (11.7 ug/ml), 100mM in each of sodium acetate, Mes, and Tris-HCl, and purified APP (79 nM). At t=0 and 3 hr, aliquots (15 ul) were removed mixed with 7.5 ul of 3X sample buffer and subject to immunoblot analysis with the C-terminal polyclonal antiserum according to Example 8. The 28, 18 and 14 kDa reaction products are located with arrows.

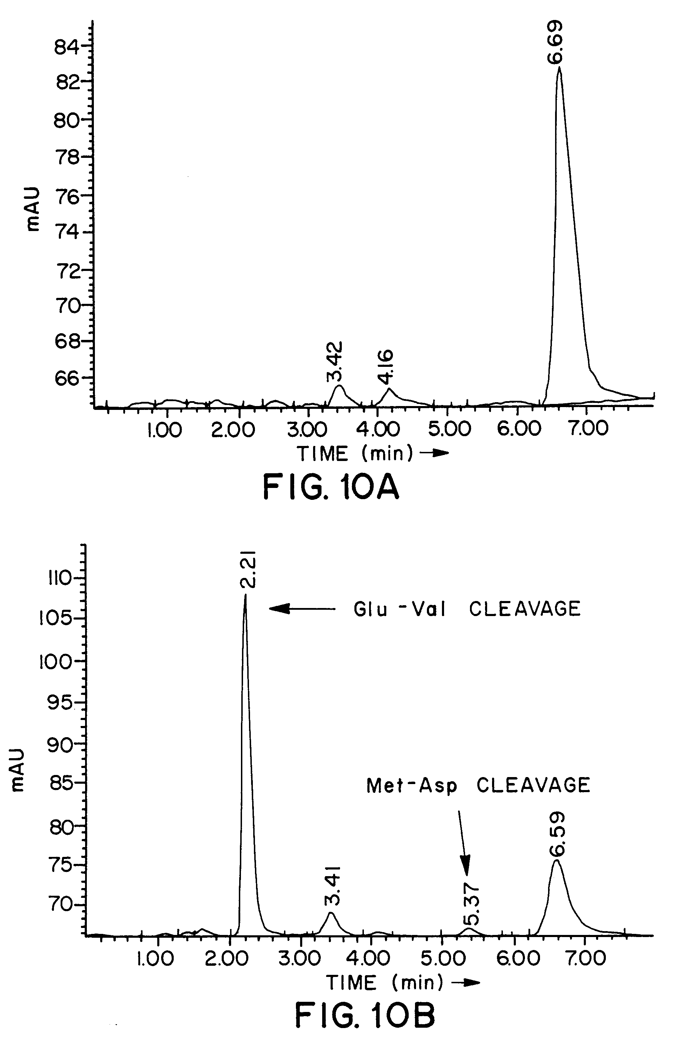

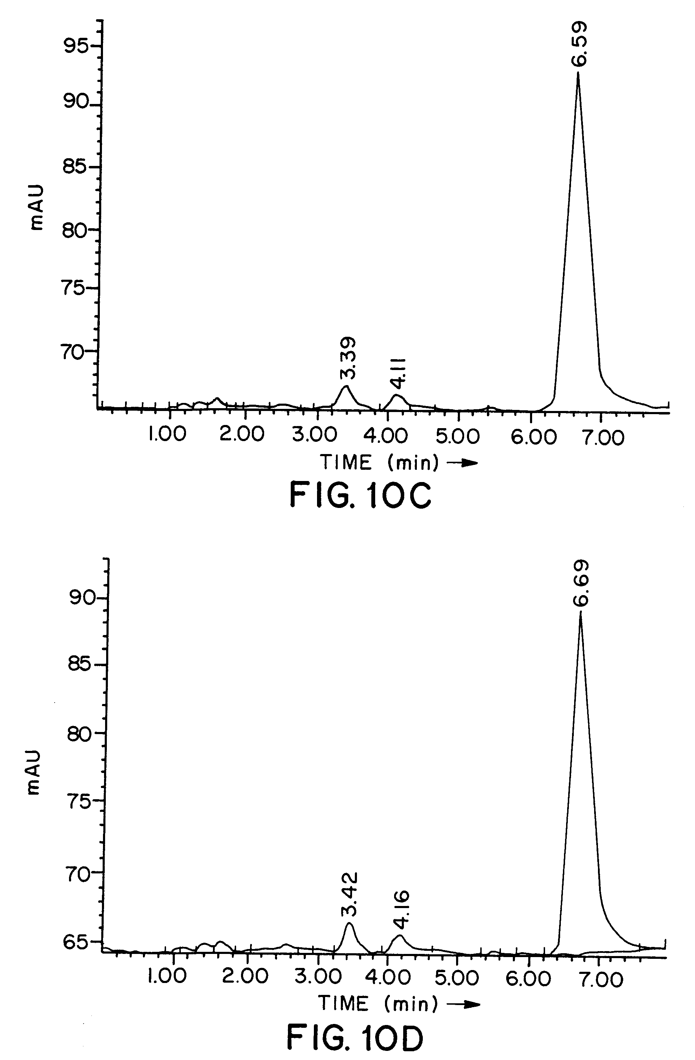

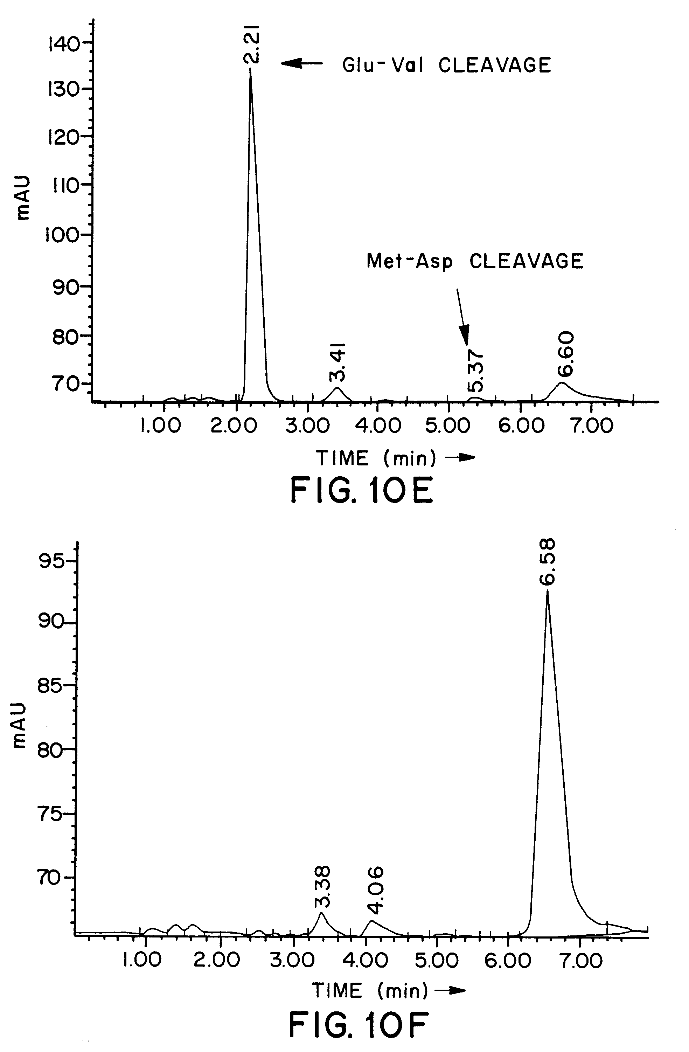

**Figure 10 shows cleavage of N-Dansyl-Ile-Ser-Glu-Val-Lys-Met-Asp-Ala-Glu-Phe-Arg-NH2 (SEQ ID NO: 7) by cathepsin D and the P-2 enzyme (peak B, Figure 5) following further purification on Superose 6HR.**

Reactions (30 ul) were initiated at 37 C by enzyme/inhibitor addition thereby achieving the following t=0 component concentrations: Cathepsin D (2.8 ug/ml), or P-2 enzyme from figure 6 (17.5 ug/ml), N-Dansyl-peptide (24 uM), captopril (0.3 mM) in a cocktail buffer comprising 130 mM in each of acetate, Mes and Tris pH 5.0. Samples contained either pepstatin A (213 uM in 3% v/v final methanol) or an equivalent final concentration of methanol only. At 0, 2, 4, 8 and 24hr, reactions were terminated by addition of 12 % (v/v) TFA (10 ul) and subject to HPLC analysis according to example 2 and 3. Representative traces are shown for: P-2 enzyme, t=0 hr (panel A); P-2 enzyme, t=24 hr (panel B); P-2 enzyme plus pepstatin A, t=24 hr (panel C); cathepsin D, t=0 hr (panel D); cathepsin D t=24 hr (panel E); cathepsin D plus pepstatin A, 24 hr (panel F).

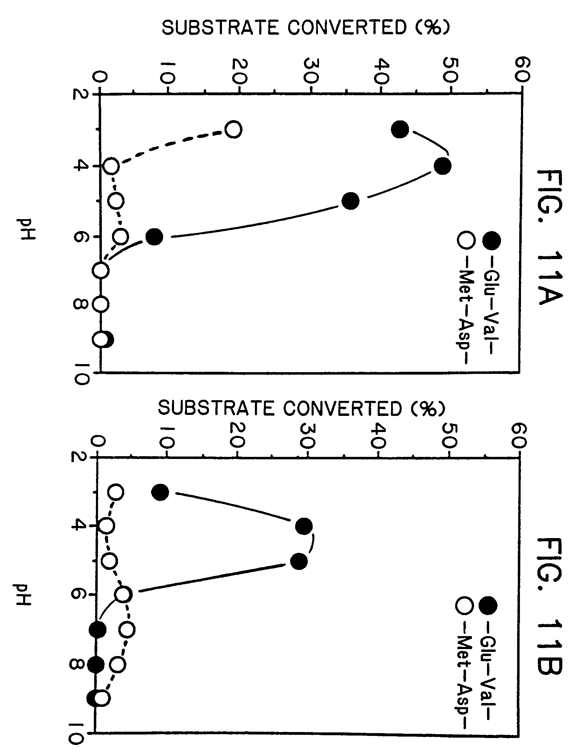

**Figure 11 depicts pH dependence of N-Dansyl-Ile-Ser-Glu-Val-Lys- Met-Asp-Ala-Glu-Phe-Arg-NH2 (SEQ ID NO: 7) cleavage by cathepsin D and the P-2 enzyme (peak B).**

Reaction conditions were essentially as described in Figure 10, except that the cocktail buffer was adjusted to the indicated pH values. (a) cleavage by cathepsin D, and (b) cleavage by P-2 enzyme. Rates of cleavage at the -Glu-Val- (closed circles) and -Met-Asp- (open circles) bonds are shown in each case.

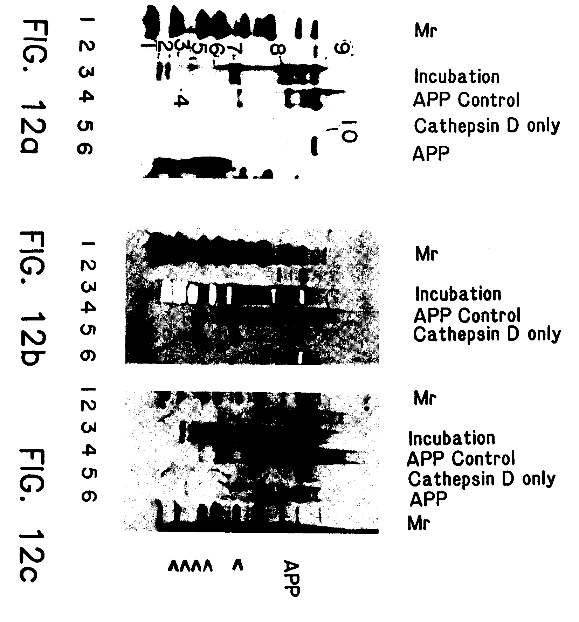

**Figure 12 is SDS-PAGE analysis of reaction products from the preparative digestion of APP by cathepsin D.**

Panel (a) is a photograph of the coomassie stained electroblot prior to excision of bands, panel (b) is the corresponding blot after band excision, and panel (c) is the corresponding immunoblot analysis (1/100 dilution of monoclonal 286.8A) of a parallel series of reactions to those depicted in panels (a) and (b). Reactions in (a) were initiated at 37°C by substrate addition thereby achieving the following initial component concentrations: APP (15.6 uM), cathepsin D (0.17 uM, 7.145 ug/ml), in 40 mM sodium acetate pH 5.0, containing 30 mM NaCl. At t=16 hr the reaction mixture was concentrated to 15 ul by speed vac, mixed with 7.5 ul of 3X sample buffer and subject to SDS-PAGE. Reactions in (c) were performed in essentially the same manner except that the APP concentration was decreased to 3.2 uM. For both experiments (in a and c), incubations were performed with the complete incubation system (lanes 3), and in the absence of cathepsin D (lane 4, cathepsin D added back immediately after addition of the sample buffer). Lane 5 in each case contained cathepsin D only controls, while lane 6 contain a purified APP as migration standard. Prestained molecular weight markers are in lane 1. In (c), the main immunoreactive products are located with arrows.

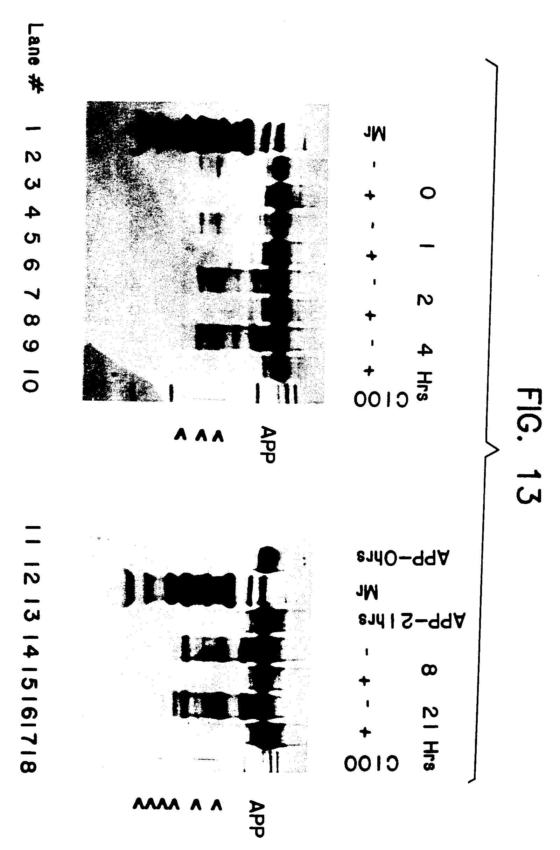

**Figure 13 shows a time course of cathepsin D catalysed APP degradation monitored using a monoclonal antibody to the N-terminus of beta-amyloid.**

Reactions were initiated at 37°C by APP addition thereby achieving the following initial component concentrations: APP (448 nM), cathepsin D (30 nM), and when included pepstatin A (97.2 μm) in 83 mM sodium acetate buffer pH 5.0. At the indicated time points, aliquots (20 ul), were removed mixed with 10 ul of 3X SDSPAGE sample buffer and subjected to immunoblot analysis using monoclonal antibody 286.8A (1/100). Reactions were performed in the absence (-) or presence (+) of pepstatin A (delivered in methanol). All samples received 2.7 % (v/v) methanol. Lanes 1 and 12 contained prestained Mr markers, Lanes 10 and 18 contained C-100 Mr marker. Lanes 11 and 13 contained APP incubated without cathepsin D for zero and 21hr respectively. The main product fragments are indicated with arrows.

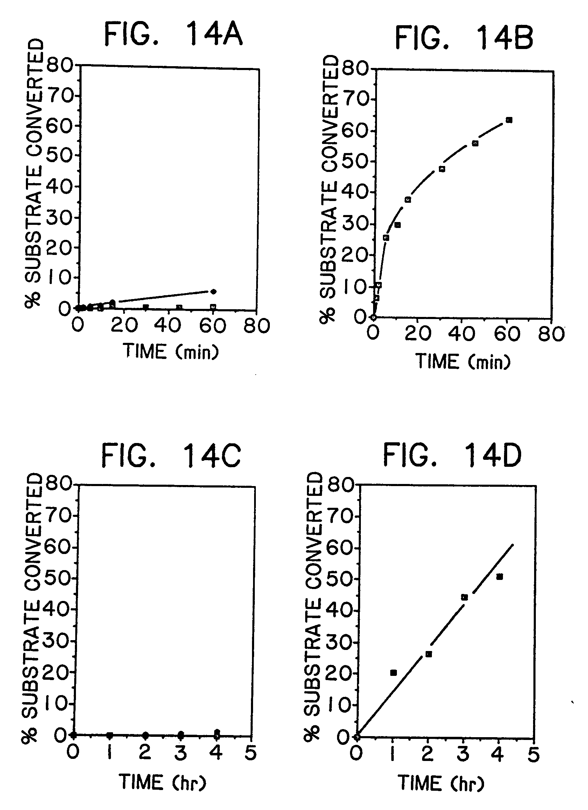

**Figure 14 shows the effect of amino acid substitution on the time course of hydrolysis of synthetic peptides by cathepsin D and the P-2 derived enzyme (peak B).**

Reactions were initiated at 37 C by substrate addition to achieve the following initial component concentrations: Cathepsin D (2.5 ug/ml) or P-2 peak B enzyme (7.5 ug/ml); N-dansyl-peptide (58 uM), captopril (0.3 mM), with or without pepstatin A (213 uM), sodium chloride (75 mM), in 135 mM buffer in each of tris, Mes and acetate buffer pH 5.0. At various times, aliquots (30 ul) were removed, adjusted to 12.5% (v/v) in TFA and subject to RP-HPLC analysis. Time course of hydrolysis for the following substrate/protease combinations are shown:
a) N-dansyl-Ile-Ser-Glu-Val-Lys-Met-Asp-Ala-Glu-Phe-Arg-NH2 (SEQ ID NO: 7) by cathepsin D;
b) N-dansyl-Ile-Ser-Glu-Val-Asn-Leu-Asp-Ala-Glu-Phe-Arg-NH2 (SEQ ID NO: 3) by cathepsin D;
c) N-dansyl-Ile-Ser-Glu-Val-Lys-Met-Asp-Ala-Glu-Phe-Arg-NH2 (SEQ ID NO: 7) by the P-2 enzyme (peak B, Figure 5 following gel filtration, Figure 6);
d) N-dansyl-Ile-Ser-Glu-Val-Asn-Leu-Asp-Ala-Glu-Phe-Arg-NH2 (SEQ ID NO: 3) by the P-2 enzyme (peak B, Figure 5).
Cleavage at the E-V bond (squares, panel A and C), M-D bond (closed diamonds, A and C), or so as to generate the metabolite at retention time 4.4 min (in B and D) are shown.

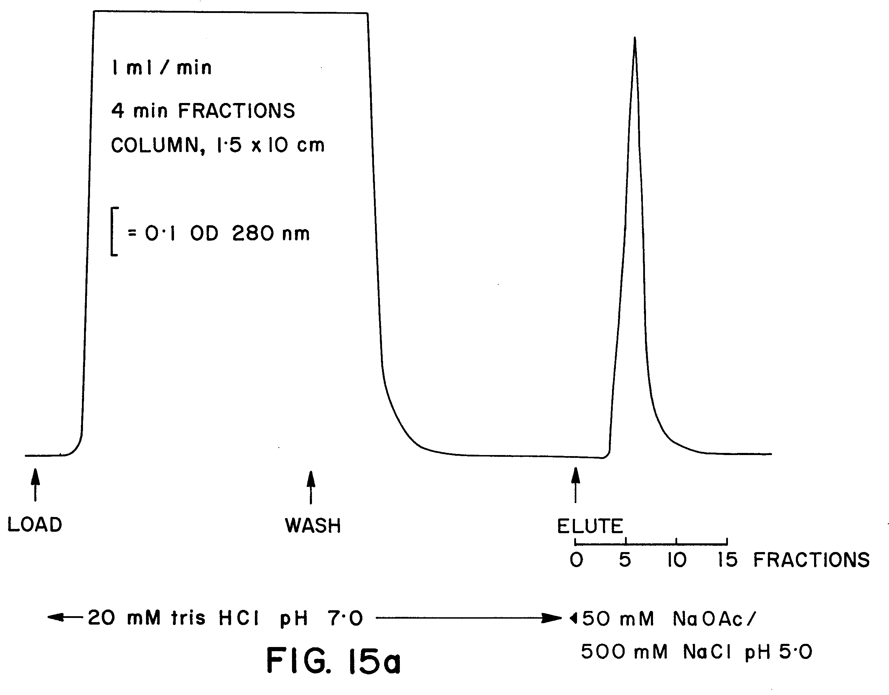

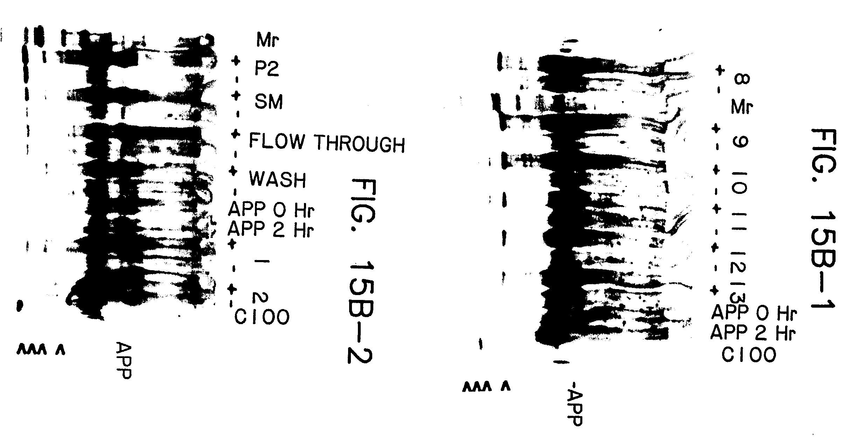

**Figure 15 shows purification of solubilized P-2 fraction on aprotinin sepharose.**

Experiment was perfromed according to Example 1. (a) is typical A280 nm elution profile, and (b) is an immunoblot assay (rabbit antiserum to C-terminal domain of APP) for APP processing activity in the eluted fractions. The arrows indicate the migration of the main APP degradation products. Note the appearance of breakdown products in fractions 8-13 from acid elution.

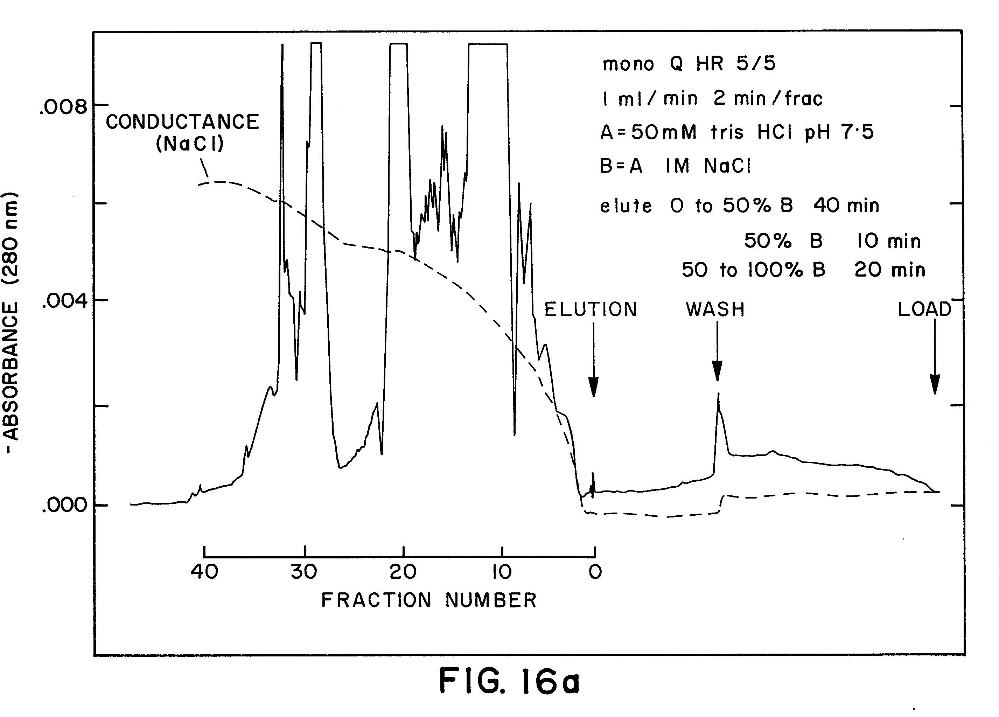

**Figure 16 shows purification of P-2 derived aprotinin binding protease on mono-Q.**

(a) shows A280 nm elution profile, (b), activity of eluted fractions using a rabbit anti-C-terminal APP antiserum (1/1000 dilution) for detection, and (c), activity of eluted fractions using Mab 286.8A.(1/100 dilution of 1.6 mg/ml pure IgG) for detection. The three arrows indicate the migration of the 11, 14 and 18 kDa C-terminal APP degradation products.

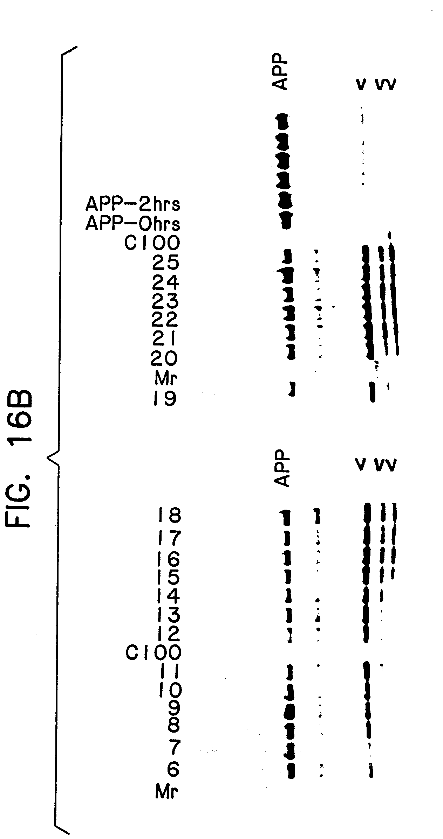

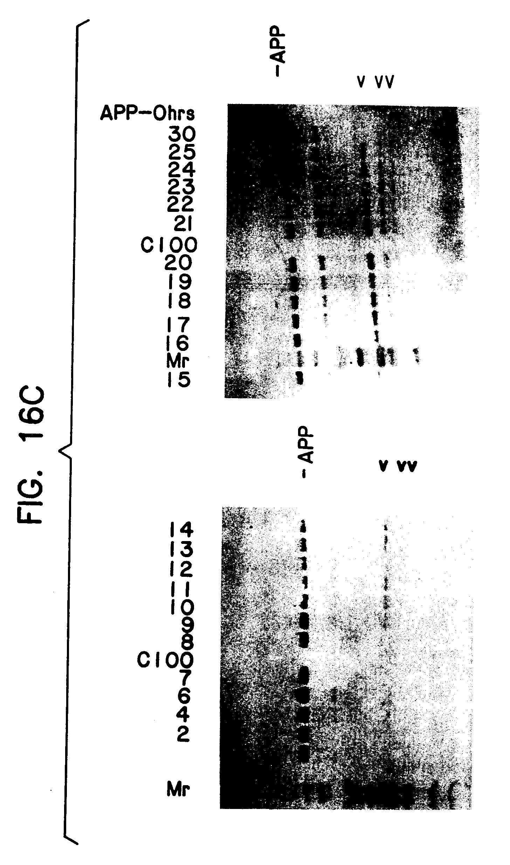

**Figure 17 shows pH and ionic strength dependence of APP degradation catalysed by the pool Y serine protease.**

Panel (a) shows pH dependence. Reactions were initiated at 37°C to achieve the following t=0 component concentrations: Pool Y protease (5 ul of fraction 16 from Figure 16), APP (38 nM), in a cocktail buffer comprising 32 mM in each of acetate, Mes and Tris adjusted to the indicated pH values. Reaction mixtures (16 ul) were terminated after 2 hr by the addition of 7.5 ul of 3X sample buffer. Immunoblots were developed essentially as described in Example 8, using a rabbit polyclonal antiserum to the APP C-terminal domain. Lanes 1 and 12 contain prestained Mr markers. Lane 9 contained C-100 and Lanes 10 and 11 contain APP incubated for 0 and 3hr respecively at pH 6.5 in the absence of pool Y. Panel (b) shows ionic strength dependence. Reactions were performed essentially as described in (a) except the buffer was 95 mM Mes pH 6.5 containing the indicated molar concentrations of sodium chloride. APP cleavage in the absence (lanes 2 to 7) or presence (lanes 9 to 14) of pool Y are shown for each concentration of sodium chloride. Lane 1 and 8 contain Mr markers and C-100 standard respectively. The arrows indicate the migration of the 11, 14 and 18 kDa APP fragments.

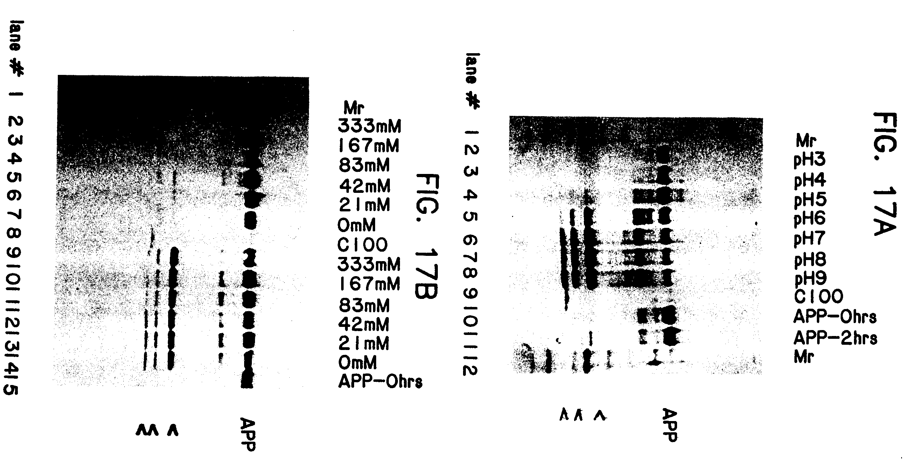

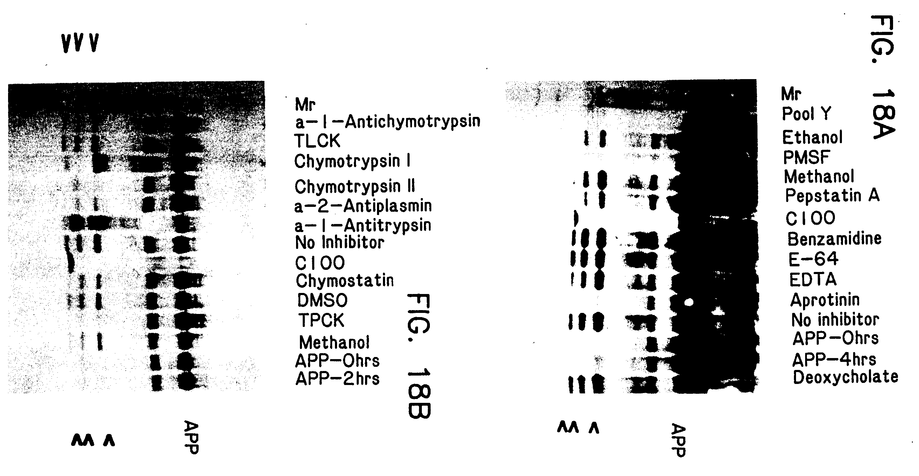

**Figure 18 depicts inhibitor selectivity of the pool Y protease.**

Reactions were initiated at 37°C by enzyme addition to achieve the following initial component concentrations in a 16 ul volume: pool Y # 3-5 (14 ug/ml) after purification on a superdex 75 column, APP (35 nM), in 30 mM Mes buffer pH 6.5. Reactions were terminated by addition of 7.5 ul of 3X sample buffer. Immunoblots were developed using the rabbit antiserum to the APP C-terminal domain according to example 8. Data are shown for the effect of the following inhibitors: Panel (a) 860 uM PMSF in methanol (lane 4), 400 uM pepstatin in methanol (lane 6), 5 mM benzamidine (lane 8), 350 uM E-64 (lane 9), 7.7 mM EDTA (lane 10), 15 uM aprotinin (lane 11), and 0.1 % (w/v) deoxycholate (lane 15). The following controls were also run: no inhibitor (lane 12), ethanol (lane 3), methanol (lane 5), pool Y only (lane 2), APP only at time zero (lane 13) and 4 hr (lane 14). Lanes 1 and 7 respectively show prestained Mr markers and the C-100 standard. Panel (b) 1.8 uM alpha-1-antichymotrypsin (lane 2), 156 uM TLCK (lane 3), 46 uM chymotrypsin inhibitor I (lane 4), 119 uM chymotrypsin inhibitor II (lane 5), 4 uM alpha-2-antiplasmin (lane 6), 51 uM alpha-1-antitrypsin (lane 7), 98 uM chymostatin administered in DMSO (lane 10), 153 uM methanolic TPCK (lane 12). Controls included: no inhibitor (lane 8), DMSO (lane 11), and methanol (lane 13). Pre-stained molecular weight markers and C-100 standards were applied to lanes 1 and 9 respectively. Incubation times of 4 and 2 hr were used in panels a) and b) respectively. The arrows indicate the migration of the 11, 14 and 18 kDa APP degradation products.

## DETAILED DESCRIPTION OF THE INVENTION

### Example 1. Human Brain Protease Isolation.

Two general approaches were taken for protease isolation. In the initial studies brain protease activities were isolated as described under "Method 1", below. Characterization of the resultant enzyme activities obtained from ion-exchange chromatography is described in Examples 3 and 8 and lead to the identification of six different activities which were able to degrade recombinant CHO cell derived APP (Example 8) but were relatively innactive as peptidases (Example 3).

One of the six activities was subsequently identified as cathepsin D (Example 9), and was further characterised according to its chromatography on gel filtration.

In an alternate approach to attempt to affinity purify some of the human brain serine proteases described in Example 8 (Table 4), "Method 2" was implemented. This procedure was based on the affinity purification of serine proteases using aprotinin sepharose at an early step, and lead to the identification of a serine protease(es) (Example 10), which also exhibited the capacity for APP C-terminal processing.

**Method 1:**

**i) Sub-cellular fractionation.** Sections of human frontal cortex pole 9 region (4.5g) from four separate, age-matched Alzheimer's patients were weighed out while frozen (-70°C), added to 150 ml of 0.32M sucrose at 4°C and scissor minced. The suspension was homogenized in batches using a 100 ml Elvehjem glass teflon potter (10 return strokes). The combined homogenate was centrifuged (1000 g x 10 min) in a Sorval SS-34 rotor.

The loose pellet was removed, re-homogenized and centrifuged as described above. The supernatant for each extraction was combined and centrifuged at 15,000 g x 30 min in the Sorval SS-34 rotor. The resultant "P-2" pellet was resuspended in 100 ml of ice cold 0.32M sucrose by vortexing and stored at -70°C. The supernatant from the last spin was centrifuged at 105,000 g x 60 min to yield the supernatant or soluble fraction ("S"), and the microsomal fraction ("M") which was resuspended in 60 ml of 0.32 M sucrose. Both S and M were stored at -70°C.

### Table 1

| Summary of protein recoveries: | | | |
|---|---|---|---|
| Fraction | Volume (ml) | Control (mg) | AD (mg) |
| Soluble (S) | 250.0 | 315.0 | 472.0 |
| P-2 pellet | 100.0 | 436.0 | 412.0 |
| Microsomal (M) | 60.0 | 105.0 | 89.4 |

**ii) Solubilization.** The membranous control or AD subfractions (P-2 or M) were solubilized by adjusting to the following conditions: 2% (v/v) Triton X-100 containing 50 mM Tris HCl buffer, pH 7.5. After stirring at 4°C for 3.5 hrs, the suspensions were centrifuged at 105,000 g x 60 min in a Beckman 70 Ti rotor. The following final protein concentrations were used in solubilization, for P-2 (3.9 to 4.0 mg/ml); and for M (1.4 to 1.6 mg/ml). Solubilized supernatants were stored at -70°C for later use. The soluble fraction was not treated with detergent but rather was adjusted to 50 mM in Tris HCl, pH 7.5, by the addition of stock 1M buffer.

**iii) Ion-exchange chromatography.** Chromatography was performed using a Gilson gradient liquid chromatograph (model 305 and 306 pumps) equipped with a 50 ml Rheodyne stainless steel loop injector model 7125, and connected to a Mono Q HR 10/10 column (Pharmacia, Piscataway, NJ). Absorbance of column effluent was monitored at 280 nm using a Pharmacia UV-M detector and a Kippen-zonen chart recorder.

Protein fractions of P-2, microsomal (M), or soluble (S) were loaded onto the column and equilibrated with 50 mM Tris HCl, pH 7.5 (conductivity 1.8 mU at 4°C) at a flow rate of 2 ml/min. The column was then washed with the equilibration buffer until the A280 nm in the eluent decreased to zero whereupon the column flow rate was increased to 4 ml/min.

Proteins were eluted as follows:

Solvents:   A = 50 mM Tris HC1 pH 7.5

B = 50 mM Tris HC1 pH 7.5, containing 1M NaCl.

Program:   0 - 50 % B over 70 min

hold 50 % B for 10 min

50 - 100 % B over 10 min

hold 100 % B for 10 min

re-equilibrate.

Four mililiter fractions were collected throughout chromatography.

The following protein loads were applied per column run:

P-2, 97 mg (control) and 95 mg (AD);

S, 50mg (control) and 68 mg (AD); and

M, 36 mg (control) and 31 mg (AD).

In the initial studies, eluted fractions were monitored for A280 nm, total protein (Bradford assay), and peptidase activity (as decsribed in Examples 2 and 3). Pools made on the basis of peptidase activity were then prepared (Example 3) and then tested for their capacity to process CHO cell derived APP C-terminally (described in Example 8).

In all further studies however eluted fractions were also individually tested for their capacity for C-terminal processing of recombinant APP derived by baculo virus directed expression (Example 9).

**iv) Gel filtration chromatography.** A typical example of gel filtration is depicted in Figure 3. More generally, chromatography was performed as described below. Mono-Q fractions from the purification of P-2, and containing APP C-terminal processing activity were pooled, concentrated to less than 0.25 ml and applied to a tandem arrangement of two Superose 6HR columns equilibrated with 10 mM Tris HCl buffer pH 7.5 and containing 150 mM NaCl. A flow rate of 0.3 ml/min was used througout. Fractions were monitored for A 280 nm, total protein (Bradford assay), peptidase activity, and activity for C-terminal processing of recombinant APP.

**Method 2:**

**i) Sub-cellular fractionation.** This was performed essentially as described in Method 1, above.

**ii) Solubilization.** This was performed essentially as described in Method 1, above.

**iii) Aprotinin-sepharose chromatography.** Soluble (230 mg), P-2 (216 mg) or microsomal fraction (47 mg) described above was applied to a column of aprotinin sepharose (Sigma, catalog # 42268, 1.5 X 10 cm), previously equilibrated with 20 mM Tris HCl buffer pH 7.0. Once loaded the column was washed with equilibration buffer (100 ml), and then eluted with 60 ml of 50 mM sodium acetate buffer pH 5.0 containing 500 mM sodium chloride. The flow rate was 1.0 ml/min throughout. Eluted fractions (4 ml) were monitored at 280 nm, analysed using the Bradford protein assay, and examined for APP C-terminal processing activity as described in Example 8, using recombinant APP derived by expression in a baculo virus system. Active fractions were capable of forming 11, 14 and 18 kDa (approx.) fragments which were detectable on immunoblots using an anti-APP C-terminal antibodies (see Method 6 for method of antibody production).

**iv) Ion-exchange chromatography.** Active fractions from the purification of the P-2 fraction on aprotinin-sepharose were pooled, dialyzed against 50 mM Tris-HCl pH 7.5 and applied to a mono-Q column (HR 5/5), previously equilibrated with dialysis buffer. Once loaded, the column was eluted essentially as described in Method 1. above. Active fractions (2 ml), were monitored for A 280 nm, total protein (Bradford assay), and for their capacity for baculo virus derived APP C-terminal processing. A broad peak of APP-degrading activity was observed, which was capable of forming 11, 14, and 18 kDa APP C-terminal fragments which reacted with both the APP C-terminal antibody as well as a monoclonal antibody directed to the N-terminus of the beta-amyloid peptide (see Example 6 for antibody production).

Based on the A 280 nm profile across the region containing active fractions, three pools of activity were prepared each overlapping with a distinctive A 280 mn peak. The pools comprised the following conductance ranges: pool X (12.2 to 14.4 mmho), pool Y (14.9 to 18.9 mmho) and pool Z (20.2 to 22.9 mmho).

**v) Gel filtration chromatography.** Pools X, Y, and Z were each concentrated to either 2 ml (pool X and Y) or 3.6 ml (pool z) and separately applied to a Superdex 75 column (Pharmacia, Piscataway NJ.) previously equilibrated with 50 mM Tris-HCl pH 7.5, containing 150 mM sodium chloride. Once loaded, the column was eluted with equilibration buffer. Chromatography was performed at a flow rate of 1.0 ml/min throughout. Fractions (1 ml) were monitored for A 280 nm, total protein (Bradford assay) and for C-terminal processing of baculo expressed APP. The gel filtration was calibrated by chromatography of each of the following standard proteins: Thyroglobulin (670 kDa), bovine gamma globulin (158 kDa), ovalbumin (44 kDa), myoglobin (17.5 kDa) and vitamin B 12 (1.35 kDa).

**Example 2. Peptidase assay development.**

A peptidase assay was developed to enable the high throughput detection of endoproteases in human brain tissues which might possess a specificity appropriate for APP hydrolysis at the junction between the "extracellular" domain(s) and the N-terminus of the beta-amyloid peptide region. The technology selected utilized dansylated peptide substrates, in conjunction with subsequent detection of fluorescent peptide products by RP-HPLC separation, and post column fluorescent detection.

**Evolution of peptide substrate sequence:** A fluorescently labelled dodeca-peptide substrate containing the same amino acid sequence as observed surrounding the N-terminal region of the beta-amyloid peptide sequence of human APP was prepared by solid phase peptide synthesis. Peptides were synthesized on an Applied Biosystems model 430A peptide synthesizer using Fmoc / NMP-HoBt chemistry (Fields et al., 1990, Int. J. Peptide Protein Res., 35:161; Knorr et al., 1989, Tetrahedron Letters, 30:1927). Usually, the peptides were cleaved and deprotected in 90% trifluoroacetic acid, 4% thioanisole, 2% ethanedithiol, and 4% liquefied phenol for 2 h at room temperature.

However the peptide: N-dansyl-Ile-Ser-Glu-Val-Lys-Met-Asp-Ala-Glu-Phe-Arg-His (SEQ ID NO: 2) was found to undergo unwanted carboxy peptidase digestion when incubated with crude tissue fractions. To attenuate carboxy peptidase digestion, the following, modified substrates were designed: N-dansyl-Ile-Ser-Glu-Val-Lys-Met-Asp-Ala-Glu-Phe-Arg -NH$_2$ (SEQ ID NO: 7) and N-dansyl-Ile-Ser-Glu-Val-Lys-Met-Asp-Ala-Glu-Phe-Arg-His-Asp-Asp-Asp-Asp (SEQ ID NO: 1).

This latter peptide was relatively insensitive to carboxy peptidase digestion even in the presence of crude tissue fractions and was used in the peptidase profiling studies of Example 3. The C-terminal alpha amide substrate (SEQ ID NO: 7) was used in the peptidase studies of Example 9 using more purified enzyme fractions. The degradation of either of the peptides was monitored using the HPLC protocol of Example 3, below.

**Example 3. Determination of peptidase activities in subfractions of normal-control and AD brains.**

**i) Incubations.** Aliquots (20 ul) of column fractions described in Example 1 were incubated with 10ul of a reaction mixture so as to achieve the following final component concentrations: N-dansyl-Ile-Ser-Glu-Val-Lys-Met-Asp-Ala-Glu-Phe-Arg-His-Asp-Asp-Asp-Asp (SEQ ID NO: 1) (50uM), captopril (300uM), in a cocktail buffer comprising 100 mM in each of: MES, Tris, and acetate, pH 6.5.

Incubation with ion-exchange fractions was performed at 37°C for 24 hrs, after which reactions were terminated by adjusting to 3% (v/v) final in TFA.

**ii) HPLC quantification of proteolytic products.** HPLC analysis was performed using a Hewlet-Packard HP1090 complete with binary solvent delivery, heated column compartment, and auto injector. Fluorescence detection (post column) was performed with an in-line Gilson model 121 filter fluorometer (excitation at 310-410 nm, emission at 480-520 nm) in conjunction with an HPLC chem-station (DOS series) and suitable software for data analysis.

Aliquots (usually 10 ul) of the above acidified incubation mixtures were injected onto a Hypersil 5uM C18 column (100 x 4.6 mm) fitted with a guard C18 5 uM guard (20 x 4.6 mm). Isocratic separation was achieved using 100 mM sodium acetate buffer, pH 6.5, containing 27% (v/v) acetonitrile. Identification and quantification of resolved metabolites was made possible by comparison with the migration of synthetic peptide products. The structure of which were confirmed by PTC-amino acid analysis and FAB-MS (See Table 2, below).

## Table 2

**HPLC retention times of known synthetic peptide standards:**

| Peptide (N-dansyl-) | HPLC retention time (minutes) | Cleavage Site |
|---|---|---|
| ISEVKMDAEFRHDDDD | 2.228 + 0.024 | Substrate |
| ISEVKM | 5.398 + 0.019 | M-D |
| ISEVK | 3.413 + 0.004 | K-M |
| ISEV | 2.692 + 0.003 | V-K |
| ISE | 2.135 + 0.002 | E-V |
| IS | 4.142 + 0.019 | S-E |

Where: A = Ala, D = Asp, E = Glu, F = Phe, G = Gly, H = His, I = Ile, K = Lys, M = Met, R = Arg, S = Ser, and V = Val.

Also note, the retention time of certain metabolites listed in Table 2, above, differ to those quoted in Example 2 for cleavage of N-dansyl-Ile-Ser-Glu-Val-Lys-Met-Asp-Ala-Glu-Phe-Arg-His (SEQ ID

NO: 2) due to variables in the HPLC set up. For example, the studies which reflect data listed in Table 2 have relatively longer retention times because chromatography was performed using a guard column in line with the HPLC column.

In all experiments the HPLC column was calibrated for day to day variation in the retention times of the enzymically generated products by analysis of synthetic product standards in parallel with the experimental samples. Data for the proteolytic metabolite profile of individual ion-exchange fractions was collected using the HP CHEM station data acquisition software.

The area under the curve for each of the six cleavage products and their retention times are stored in a peak table file. All peak tables are collated and transferred to a (6 x n) area array in EXCEL (using a custom utility written in programming language C) where n = the number of Mono Q fractions. Each row of the array represents a single peptidase analysis from a Mono Q fraction. Zeros are inserted between each column of data to artificially establish a gradient of values in the row direction.

SpyGlass takes this array and transforms it into a three-dimensional surface in which Mono Q fraction number, cleavage site and Area % for the product formed are the three axes. Contours are defined according to the following criteria set manually within the SpyGlass Program: the first contour line connects contiguous regions of the plot where 1.5% substrate conversion to the particular product was observed. Similarly successive contour lines connecting regions of 5%, 10%, 20%, 30%, 40% and 50%, substrate conversion were also displayed. The resulting contour plots represent brain peptidase maps in which fraction numbers span the ordinate, and peptide bond cleavage sites are on the abscissa, and the amount of product formed is represented by the contours.

**Results of peptidase profiling of control and AD brain subfractions.**

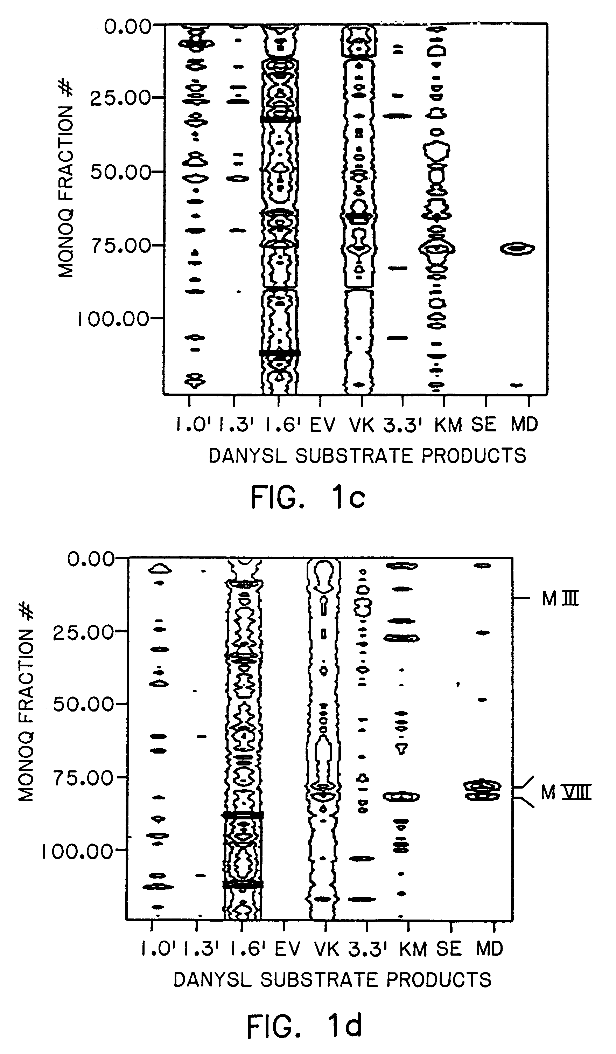

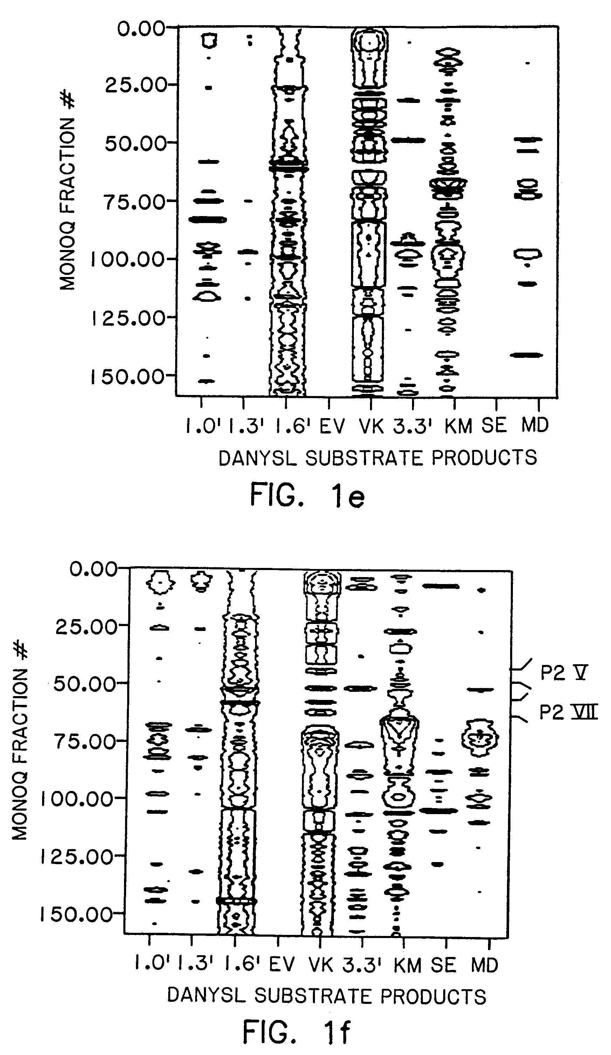

Figure 1 shows a comparison of the peptidase profiles obtained for the cleavage of the N-dansyl peptide substrate by both control and AD P-2, S, and M fractions subjected to further subfractionation by ion-exchange chromatography. The analysis enables the identification of a high number of potentially different peptidase activities throughout the subfractions of control and AD brain.

For each analysis (a to f in Figure 1), the amount of activity for cleavage at each peptide bond decreased through the series: V-K > K-M > M-D > S-E > E-V, however the K-M and M-D cleavages are of greatest interest because of their greater likelihood of representing the site of APP hydrolysis leading to C-100 formation. The metabolite recovered at 1.6 min is probably due to methionine oxidation to the sulfoxide in the substrate, as treatment of substrate with hydrogen peroxide generates a product of the same retention time (rt). The metabolites formed with rt at 1.0 and 1.3 and 3.3 min are unidentified at present.

For the K-M cleavages, overall levels of activity both in terms of the spectrum of enzymes and peak activity both for control and AD descended through the series P-2 > S = M. This was also true for the M-D cleavages in AD, wherease for the control fractions the order was S > P-2 = M.

Regarding the most abundant peptidase peaks found in AD (only those bounded by more than one contour line), the following number of obviously resolved peptidase peaks could be discriminated: **P-2**, three K-M peaks and one M-D peak; **M**, three K-M peaks and two M-D peaks; **S**, no K-M peaks and one M-D peak. Qualitative comparisons between the levels of the more abundant peaks between corresponding control and AD subfractions revealed only one noteable difference. The difference was observed in the microsomal fractions wherein the control M profile contained a single M-D product around fraction 75, wherease in the AD profile the same region clearly contained a doublet.

Because of potential variation between peptidase levels in the normal and disease state populations it is of little point to highlight quantitative differences in peptidase levels between control and AD.

In summary while it cannot be discounted that there may be qualitative or significant quantitative differences between control and AD fractions in the levels of minor peptidase forms, the overall profiles looked quite similar with only one obvious qualitative difference being apparent amongst the more abundant peaks of peptidase activity.

**iii) Consolidation of peptidase activities into discrete pools.** Based upon the peptidase Activity profiles obtained using the N-dansyl-Ile-Ser-Glu-Val-Lys-Met-Asp-Ala-Glu-Phe-Arg-His-Asp-Asp-Asp-Asp-Asp (SEQ ID NO: 1) substrate, column fractions from the ion-exchange separation of P-2, soluble (S) and microsomal (M) fraction were consolidated into contiguous pools (12 pools for M, 13 pools for P-2, and 14 pools for S). Care was taken in each case to pool the same regions of the chromatography profile both for AD and control fractions (even if no protease was detectable in the corresponding control region), and the precision of the process was checked by monitoring the conductivity of each pool using a YSI model 35 conductivity meter.

Fractions were maintained at 4°C throughout pooling and then stored at -70°C. Each peptidase pool was concentrated using an Amicon Centriprep-10 membrane. Prior to concentrating, each Centriprep membrane was washed in 50 mM Tris/HCl, pH 7.5. A 15 ml Centriprep was used for peptidase pools that contained a volume of 5 ml or more. These pools were concentrated on a Du Pont Sorval tabletop centrifuge at 2700 rpm for approximately 40 min. Pools that contained less than 4 ml were concentrated with a 2 ml Centriprep on a Du-Pont Sorval RC centrifuge (SS34 rotor) at 5000 rpm for 40 min.

After concentrating, each concentrated pool was washed with an equal volume of 50 mM Tris/HCl, pH 7.5. Pools were maintained at 4°C throughout the concentrating process and stored at -70°C. The pools are listed below in Table 3 with the conductivities and the final concentration volumes.

**Table 3.**

| Pooling and concentration of ion-exchange fractions from the separation of human brain S, M and P2 sub-fractions. | | | | | |
|---|---|---|---|---|---|
| Pool | Conductance | Control | | AD | |
| | (mmho) | (ml) | fold conc | (ml) | fold conc |
| S I | 1.6 | 0.75 | 16 | 0.75 | 16 |
| S II | 1.6 | 0.75 | 16 | 0.75 | 16 |
| S III | 5.0-6.8 | 1.50 | 13 | 0.50 | 48 |
| S IV | 7.1-7.8 | 0.75 | 16 | 0.75 | 16 |
| S V | 8.0-8.4 | 0.60 | 13 | 0.60 | 13 |
| S VI | 8.6-8.8 | 1.00 | 16 | 1.00 | 8 |
| S VII | 9.2-10.8 | 1.25 | 10 | 1.00 | 24 |
| S VIII | 11.1-11.6 | 1.00 | 16 | 0.80 | 15 |
| S IX | 12.8-13.3 | 1.50 | 13 | 1.25 | 10 |
| S X | 15.1-15.8 | 1.00 | 12 | 1.25 | 13 |
| S XI | 20.2 | 0.75 | 5 | 0.75 | 5 |
| S XII | 26.1 | 0.75 | 5 | 1.00 | 4 |
| S XIII | 33.1 | 0.75 | 5 | 0.80 | 5 |
| S XIV | 38.4 | 0.75 | 5 | 0.60 | 7 |
| P2 I | 1.6 | 13.00 | 4 | 6.00 | 9 |
| P2 II | 1.6 | 1.00 | 24 | 1.00 | 28 |
| P2 III | 1.6 | 1.00 | 36 | 0.60 | 53 |
| P2 IV | 1.6 | 1.00 | 46 | 2.00 | 24 |
| P2 V | 1.6 | 1.00 | 32 | 0.50 | 64 |
| P2 VI | 1.6-1.9 | 1.00 | 20 | 0.50 | 40 |

| | | | | | |
|---|---|---|---|---|---|
| P2 VII | 2.5-5.2 | 1.00 | 56 | 0.50 | 72 |
| P2 VIII | 5.5-7.7 | 1.00 | 16 | 0.60 | 53 |
| P2 IX | 8.0-8.6 | 0.50 | 16 | 0.50 | 24 |
| P2 X | 9.1-10.0 | 1.00 | 16 | 0.80 | 20 |
| P2 XI | 10.1-13.9 | 6.00 | 10 | 17.00 | 4 |
| P2 XII | 14.1-17.0 | 6.00 | 9 | 8.00 | 6 |
| P2 XIII | 17.1-20.8 | 1.00 | 64 | 11.50 | 5 |
| | | | | | |
| M I | 1.6 | 2.00 | 10 | 1.00 | 28 |
| M II | 1.8 | 0.60 | 25 | 0.80 | 15 |
| M III | 1.8 | 0.50 | 8 | 0.40 | 10 |
| M IV | 1.8 | 0.50 | 8 | 0.50 | 8 |
| M V | 9.7-10.2 | 0.80 | 5 | 0.50 | 24 |
| M VI | 10.5 | 0.50 | 8 | 0.50 | 8 |
| M VII | 10.9-14.5 | 1.50 | 40 | 1.00 | 56 |
| M VIII | 15.2-15.8 | 0.80 | 19 | 0.80 | 15 |
| M IX | 16.1-16.5 | 0.80 | 15 | 0.80 | 15 |
| M X | 20.7 | 0.50 | 8 | 0.50 | 8 |
| M XI | 34.5 | 0.50 | 8 | 0.50 | 8 |
| M XII | 37.7 | 0.50 | 10 | 0.50 | 8 |

## Example 4. Expression of recombinant APP 695.

This example describes the method for expressing holo-APP 695 which was then purified as described in Example 7 and then used as the recombinant substrate for the APP degradation assay described in Example's 8 through 10. Two approaches were used.

Initially, a CHO cell expression system was used to generate APP 695. Experiments using this source of APP as a substrate included the inititial activity measurements which lead to the identification of six different protease activities detectable in contigous pools of mono-Q fractions from the purification of human brain P-2, soluble and microsoaml fractions. These studies are described in Example 8.

Subsequently, recombinant APP 695 was obtained by expression using a baculovirus directed system. The greater amounts of APP 695 thereby generated made it feesible to perform the more detailed studies outlined in Examples 9 and 10, leading to the identification of certain APP degrading enzymes.

Both methods of expression are decribed below:

## Method 1: Development of a Chinese Hamster Ovary (CHO) cell line expressing holo-APP 695.

a) Vector construction. A known 2.36 Kb NruI/SpeI fragment of APP 695 cDNA from FC-4 (Kang et al., supra) was filled in by the large fragment of E. coli DNA polymerase I and blunt-end inserted into the SmaI cloning site of KS Bluescript M13+ (Stratagene Cloning Systems, La Jolla, CA.) creating pMTI-5 (App 695 under the T3 promoter). A new optimal Kozak consensus DNA sequence was then created using site-specific mutagenesis (Kunkel et al., 1987, Methods in Enzymology, 154:367-382) with the oligo: 5'-CTCTAGAACTAGTGGGTCGACACGATGCTGCCCGGTTTG-3' (SEQ ID NO: 8) to create PMTI-39. This plasmid was next altered by site specific mutagenesis (Kunkel et al., Id.) to change the valine at position 614 to a glutamate (open reading frame numbering according to Kang et al., Id.).

The full length APP cDNA containing the optimal Kozak consensus sequence and Val to Glu mutation was then cut out of PMTI-39 with NotI and a HindIII partial digest. The 2.36 Kb APP 695 fragment was then gel purified and ligated into NotI/HindIII cut pcDNAINeo (Invitrogen Corp. San Diego, CA.) to create PMTI 90 in which the APP 695 expression is placed under the control of the CMV promoter. The Val to Glu mutation was sequence confirmed and the vector used to stably transform CHO cells.

**b) Generation of stable CHO cell lines expressing APP 695 mutenes.**

Chinese Hamster Ovary K-1 cells (ATCC CCL 61) were used for transfection with the APP 695 construct. Twenty micrograms of an expression plasmid containing APP 695 and a neomycin drug resistance marker was transfected into $1 \times 10^7$ CHO cells in 0.5 ml PBS by electroporation using a Bio-Rad Gene Apparatus (Bio-Rad Laboratories, Richmond, CA.). A single pulse of 1200 V at 25 $\mu$f capacitance was administered to the cells.

Following electroporation, cells were incubated in ice for 10 minutes and collected by centrifugation. The cell pellet was resuspended in Alpha MEM, 10% fetal calf serum at a density of $5 \times 10^4$ cells/ml, and 1 ml aliquots were distributed into each well of five 24-well tissue culture cluster plates. After 48 hours incubation, cells containing the neomycin drug resistance marker were selected by addition of 1 ml of media containing 1 mg/ml Geneticin (GIBCO-BRL, Grand Island, NY.) and incubation was continued and bi-weekly changes of drug containing media.

Drug resistant cells were tested for APP 695 expression by Western blotting. Cells positive for APP 695 expression were cloned by limiting dilution, and individual clones were isolated and tested for APP 695 expression. A clone positive for APP 695 expression was subcultured and expanded into roller bottles for large scale production of APP 695 expressing cells and subsequent isolation of recombinant protein.

**Method 2: Expression of Recombinant Holo-APP 695 using Baculovirus Infected Insect Cells.**

**a ) Construction of Recombinant Vector.** The Baculovirus vector pVL1392 (Invitrogen) was cut Xba I (in the polylinker) and ligated with the gel isolated 2.36 Kb Xba I fragment from pMTI-39 (APP695 NruI/SpeI into KS Bluescript M13+ SmaI site, T3 orientation, with a new Kozak and XbaI site at the SmaI/NruI blunt fusion site). This created pMTI-103, which was transformed into DH5$\alpha$, selected on Amp, and a lithium prep of the plasmid DNA made for transfection.

**b) Cells and Virus.** Spodoptera frugiperda (Sf9) cells, purchased from the American Type Culture Collection (ATCC) were grown as suspension cultures at 28 C. in TNMFH media (Summers, M.D. and Smith, G.E. (1987) A manual of Methods for Baculovirus Vectors and Insect Cell Procedures. (Bulletin no. 1555, Texas Agric. Exp. Stn. and Texas A & M Univ., University Station, TX) containing 10% fetal calf serum. Wild type AcMNPV DNA was purchased from Invitrogen, San Diego, CA.

**c) DNA Transfection and Plaque Assays.** Foreign DNA was inserted into the genome of AcMNPV at the polyhedrin gene locus by homologous recombination by cotransfection of purified plasmid DNA (4ug) and linear viral DNA (1ug) into Sf9 cells using the calcium phosphate procedure (Summers et. al (1987) Supra). Viruses which were released by the transfected cells were purified by 2 rounds of plaque assay (Summers et. al. (1987), Supra.), where recombinant viruses were identified by visually screening for polyhedrin-negative plaques. Purified recombinant viral cultures were Subsequently screened for their ability to produce APP in infected cells by western blot analysis.

**d) Recombinant Protein Production.** 5 liter batches of Sf9 cells, grown as suspension cultures in TMNFH media containing 10% fetal calf serum at $1 \times 10^6$ cells/ml, were infected with recombinant virus at a M.O.I of 1. Cells were harvested 24 hours post infection and cell lysates prepared for purification of recombinant protein.

**Example 5. Development of expression vectors for the production of recombinant C-100 standard by transient infection of mammalian cells.**

The C-100 peptide fragment contains the C-terminal portion of APP which spans from the N-terminus of the A4 peptide to the C-terminus of full length APP (see above, BACKGROUND section). The C-100 fragment is the purported initial degradation product leading to the ultimate formation of the A4 peptide.

In one embodiment of the present invention, cell lysates from Hela S3 cells (ATCC CCL 2.2) expressing recombinant C-100 are analyzed in the immunoblot assay in parallel with the recombinant APP samples that have been incubated with brain fractions, subfractionated by Mono-Q chromatography (See Example 3). The migration and detection of the C-100 fragments serves both as a size marker for the genesis of products formed by pathologic proteases as well as a positive control for the immunodetection of C-terminal APP fragments in general.

Comparison of the size of enzymically generated products with the size of the C-100 fragment can provide insights into whether or not the enzymically generated fragments result from cleavage close to the N-terminus of the A4 peptide or alternatively within the A4 segment as would be catalyzed by secretase.

### a) Plasmid construction

Two methods were used to make plasmids for C-100 expression. Each plasmid shall be identified separately as either PMTI 73 or PMTI 100.

**PMTI 73 Construction:** The commercially available plasmid PUC-19 was digested with Ecorl to eliminate its polylinkers. Commercially available PWE16 was then inserted into the digested PUC-19 to create PMTI 2300. PMTI 2301 was derived from PMTI 2300 following BamHI/Hind III digestion using an oligonucleotide adapter. The EcoRI promoter fragment of APP was inserted into the HindIII site of pMTI 2301 by blunt end-ligation to produce PMTI 2307.

PMTI 2311 was generated-by ligating the BamHI fragment from FC-4 (Kang et al., supra) into the BamHI site of PMTI 2307. The XhoI fragment from FC-4 was inserted into the XhoI site of PMTI 2311 to generate PMTI 2312. PMTI 2323 was generated by insertion of the 2.2 kb BglII/EcoRI fragment from the EcoRI genomic clone of the mouse metallothionein-I gene into the ClaI site of PMTI 2312. To generate minigene PMTI 2337, the sequences between the KpnI and BglII sites of PMTI 2323 were deleted and the clone was ligated using synthetic oligonucleotide adaptor, sp-spacer-A4.

PMTI 2337 was cut with Bam H1/SpeI and the fragment ligated into the Bam H1/Xba1 restriction sites of bluescript KS (+) (Stratagene) to create PMTI 2371. PMTI 2371 was cut Hind III/NotI to release a 0.7 kb fragment coding for the terminal 100 amino acids of APP 695. Also encoded was the sequence for signal peptide. This insert was ligated into the Hind III/NotI site of pcDNAINEO (Invitrogen Corp.) to create the plasmid PMTI 73.

**PMTI 100 Construction:** PMTI 90 (see Example I) was cut XbaI/HindIII to release a 0.6 kb fragment again coding for the terminal 100 amino acids of APP 695 and this was ligated to the XbaI/HindIII site of pcDNAINEO to create PMTI 100. In each case vectors,inserts and plasmids were purified by methods known to those skilled in the art.

**b) Transfection and expression of C-100 Fragment.** Preparation for small scale expression of C-100 standard was initiated by seeding $5 \times 10^5$ cell Hela S1 cells in each well of a 6 well costar cluster (3.5cm diameter) 24 hours before use.

Sufficient vaccinia virus vTF7-3 was trypsin treated to infect at a multiplicity of 20 plaque forming units per cell, mixing an equal volume of crude virus stock and 0.25 mg/ml trypsin, then vortexed vigorously. The trypsin treated virus was incubated at 37°C for 30 minutes, with vortexing at 10 minute intervals. Where clumps persisted, the incubation mixture was chilled to 0°C and sonicated for 30 seconds in a Sonicating water bath. The chilled sonication was repeated until no more clumps were detected.

The trypsin treated virus was then diluted with sufficient serum free DMEM for each well with Hela S1 cells to have 0.5 ml of virus. Medium was aspirated way, then the cells were infected with virus for 30 minutes, with rocking at 10 minutes intervals to distribute the virus.

Approximately 5 minutes before infection was ceased, fresh transfection mixture was prepared as follows: To each well was added 0.015 ml lipofection reagent (Betheseda Research Labs, Gathersburg, MD.) to 1 ml OPTIMEM (Betheseda Research Labs, Gathersburg, MD.) in a polystyrene tube, mixing gently. Vortex was avoided. Then, 3 μg CsC1 purified DNA was added and mixed gently.

Virus mixture was aspirated from cells, then the transfection solution was introduced. The resulting mixture was incubated for three hours at 37°C. Each well was then overlaid with 1 ml of OPTIMUM and incubated at 37°C in a $CO_2$ incubator overnight.

Cells were harvested at 20 hours post transfection by centrifugation, and lysates were prepared on ice with the addition of 0.2ml of a lysis buffer which contained 1% Triton X-100, 10 μg/ml BPTI, 10 μg/ml Leupeptin, 200 mM Nacl, 10 mM HEPES, 1 mM $CaCl_2$, 1 mM $MgCl_2$, 1 mM EDTA, adjusted to pH 7.5. Complete lysis was monitored by light microscopy, and harvested immediately. Lysis took less than 1 minute to complete, with delay at this step causing lysis of nuclei resulting in a gelatinous mess.

Recombinant lysates were stored at -20°C for later use. Preferably, recombinant lysates should be diluted 1:50, in (3X) SDS PAGE sample buffer which is devoid of 2-mercapto ethanol prior to freezing.

A comparison of the size of the proteins produced by expression with either PMIT 73 or PMTI 100 using SDS-PAGE/ immunoblot with the APP C-terminal antibody was performed (Figure 2e). The study showed that PMTI 100 directed the expression of a single immunoreactive band, wherease, PMTI 73 directed the expression of two major bands of similar molecular size. A less intense band of intermediate. size was also evident in PMTI 73 when applied to gels in higher amounts (Figures 2a-2d).

Application of the PMTI 100 protein to SDS PAGE gels in higher amounts results in the appearance of a series of fainter bands (eg. Figure 2d). Besides an intense band of C-100 monomer of apparent Mr 11.7 kDa, fainter bands are observed at Mr 25.5 kDa, 35 kDa and 45 kDa, which are attributed to the formation of dimeric, trimeric and tetrameric aggregates, respectively, of the C-100 monomer. An additional faint band of Mr 18.9 kDa is also observed. Similar phenomena have been reported in the literature with similar interpretations (Dyrks et al., 1988, EMBO J. 7:949).

The largest of the three bands produced by PMTI 73 was slightly larger than the single band observed with PMTI 100. Amino acid sequence analysis of the largest band from PMTI 73 expression showed that the signal peptide sequence was cleaved from the initial translation product to yield a C-100 fragment containing 5 extra amino acids at the N-terminus.

## Example 6. Production of immunochemical reagents.

Three different immunochemical reagents were used in the studies of the present invention:

i) A Rabbit polyclonal antiserum which recognized the C-terminus of APP was obtained and used for immunoblot detection of C-terminal APP fragments generated by proteolytic processing according to the assay conditions described in Example 8;

ii) An affinity purified antibody which recognized the C-terminus of APP was prepared and used to synthesis an immunoaffinity column for the affinity purification of APP expressed in a baculo virus directed system (see Example 7); and

iii) a mouse monoclonal antibody which recognizes the N-terminus of the beta-amyloid peptide was generated and used in an immunoblot assay to determine whether C-terminal APP fragments generated by proteolytic digestion of holo-APP 695 contained the full length beta-amyloid peptide (see Examples 9, and 10 for specific applications).

The method of generation of each of the three immunochemicals is presented below.

**i) Rabbit polyclonal antiserum to the C-terminus of APP.** Antisera were elicited to the C-terminal domain of human APP 695, and were prepared in accordance with the method as described in Buxbaum et al., 1990, Proc. Nat'l. Acad. Sci. 87:6003-6006. A synthetic peptide (hereinafter "β APP 645-694") corresponding to the COOH-terminal region of APP 695 was obtained from the Yale University, Protein and Nucleic Acid Chemistry Facility, New Haven, CT.

β APP 645-694 was used to immunize rabbits to elicit polyclonal antibodies. Sera were screened by immunoblot analysis of lysates of E. coli that expressed a fusion protein including the amino acids 19 through 695 of human APP 695. Sera which were immunoreactive against the recombinant fusion protein were further screened for immunoprecipitating activity against [$^{35}$S] methionine-labeled APP 695, which was produced from B APP 695 cDNA by successive in vitro transcription (kit purchased from Stratagene, La Jolla, CA) and translation (reticulocyte lysate kit purchased from Promega Corp., Madison, WI).

**ii) Polyclonal antibody affinity column for the purification of holo-APP.**

Purification of Synthetic APP C-terminal Peptide Immunogen. 80-90 mg of crude synthetic peptide (P-142) spanning the C-terminus of APP(649-695) with a Cysteine residue at the N-terminus was purified by HPLC (yield 42%;34mg).Amino acid analysis, N-terminal sequence analysis and Laser Desorption Time of Flight Mass Spectrometry showed the purified peptide to be a mixture of full length and N-terminally truncated peptides (2/1 full length to truncated).

Immunization of Rabbits with Purified P-142 immunogen. The HPLC purified peptide APP(649-695) was used to immunize rabbits. Two rabbits each received an initial challenge with 125 ug of peptide in complete Freunds Adjuvant followed by subsequent boosts of the same amount of peptide in incomplete Freunds Adjuvant at three week intervals. Fourteen bleeds were collected over a 9 month interval and optimal production of Ab was observed for bleeds at 16 thru 32 weeks (shown by western analysis with Vacinnia C100 and CHO APP). Bleeds in this interval were pooled for an approximate volume of 90-100mls of anti-sera.

Preparation of an immobilized APP649-695 affinity matrix for purification of antisera. 9.7 mg of purified peptide APP(649-695) was coupled to maleimide activated BSA using the Pierce Imject activated Immunogen Conjugation kit with BSA. About 40% of the Peptide (3.88mg) was coupled to BSA as determined by Ellman's Reagent. The BSA coupled peptide was separated from uncoupled peptide by gel filtration (Purification buffer from kit= 83mM NaH2PO4 pH 7.2;900mM NaCL). The pooled void volume from the gelfiltration column (2.9mg P-142 conjugated to BSA/12.5mls) was coupled to 1gm(3.5mls) of CnBr activated sepharose (>90% peptide conjugate coupled by standard Pharmacia protocol). Remaining sites were blocked with Ethanolamine. The sepharose affinity matrix was packed into a 1.0 x 3.5cm glass column.

Purification of Rabbit Polyclonal Antibody using the APP(649-695) affinity column. The combined rabbit anti-sera from bleeds of optimal Ab production were pooled (100mls/3.9gms protein) and diluted 1:1 (v/v) with wash buffer (100mM NaHC03 pH8.3;750mM NaCl) and loaded onto the peptide affinity column at 1.0ml/min at 4°C. After loading (200mls), the column was washed with wash buffer (75ml) until A280 returned to zero.The IgG was eluted with 100mM Glycine pH2.5 (40ml). One minute fractions were collected into tubes containing 100ul of 1.0M Tris HCl pH8.0. The neutralized low pH IgG eluant was pooled (35mls;14.7mg) and dialyzed against 1.0 liters of 100mM NaHCO3 pH8.3;500mM NaCl at 4°C.

Preparation of Immunoaffinity column: Coupling purified Rabbit IgG to Sepharose. 5.0 gms of CnBr Sepharose was activated with 50mls of coupling buffer (100mM NaHCO3 pH8.3;500mM NaCl) and mixed with dialyzed IgG pool on an orbitron for 21 hrs at 4°C. After coupling, the resin was rinsed 1X with coupling buffer through a sintered glass filter, followed by 3x rinses with 100 ml ea of blocking buffer.(100mM NaHCO3 pH8.3;500mM NaCL;1.0M Ethanolamine. Two succesive intermediate rinse steps with coupling buffer (100mls), then low pH buffer (100mM NaOac pH 4.0; 500mM NaCl(100ml) and a final rinse with coupling buffer (100ml) completes the resin preparation. The coupling was 87% for a total of 17.5 mls of resin. (0.727mg IgG/ml res-in).

### iii) Generation and epitope mapping of a monoclonal antibody to the beta-amyloid peptide.

Hybridoma Methodology. Balb/c mice were immunized by multiple injections of a mixture of the following two synthetic peptides: 1) APP amino acids 597 to 638 of holo-APP695 (numbering according to Kang et al., Id.) containing beta amyloid, and 2) APP695 amino acids 645-695 containing the C-terminal domain. Splenocytes from immunized animals were fused with X63/Ag 8.653 mouse myeloma cells using standard procedures (Herzenberg et al., 1978, In: D.M. Weir (Ed.), Handbook of Experimental Immunology, pp 25.1-25.7, Blackwell Scientific Publications, Oxford, UK). Supernantants from the resultant hybrids were tested for the presence of anti-peptide specific antibodies using an EIA in which the beta amyloid peptide immunogen was bound to the microtitre plate. Cultures secreting antibody which reacted with the synthetic peptide used as immunogen were cloned twice by limiting dilution, and their isotype determined as described (Wunderlich et al., 1992, J. of Immunol. Methods, 147:1). Secreted IgG was purified from the serum free fermentation broth of cloned hybridoma cells by protein-A affinity chromotography of the spent culture fluid.

Epitope Mapping. One of the anti-peptide monoclonal antibodies, an IgG 2b designated C286.8A, gave good reactivity with synthetic beta-amyloid peptide both by EIA as well as by immunoblot assay. The epitope reactivity of the monoclonal was determined using a competitive EIA. Synthetic peptides containing amino acids 597-612, 597-624, 597-638, 608-624, 621-631 and 645-695 of human APP695 (numbering according to Kang et al., Id.) as well as N-dansyl-Ile-Ser-Glu-Val-Lys-Met-Asp-Ala-Glu-Phe-Arg-His-Asp-Asp-Asp-Asp (SEQ ID NO: 1) were tested for the ability to block binding of C286.8A to APP 597-638.

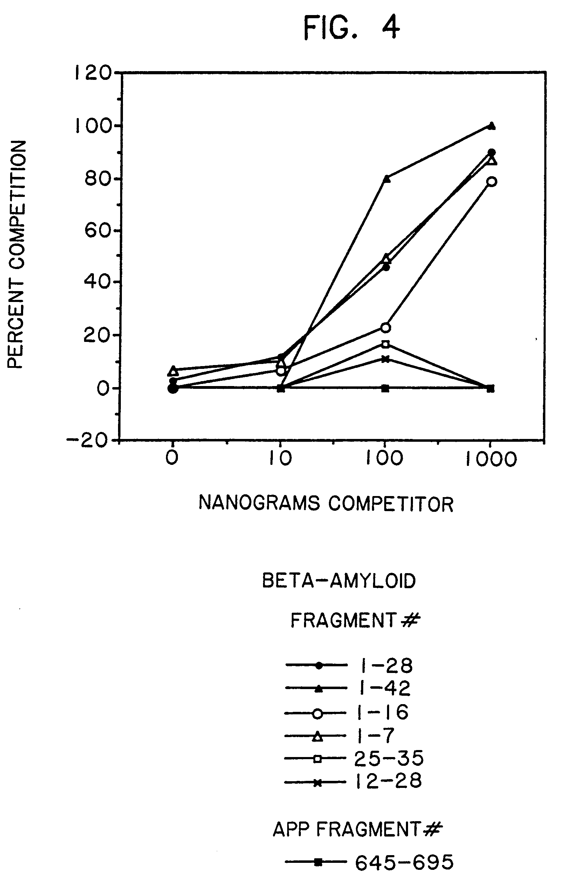

Peptides which are recognized by the antibody will, if preincubated with the antibody in solution, deplete the solution concentration of the antibody available for subsequent reaction with beta-amyloid peptide bound to a microtitre plate. The result of such an experiment is shown in Figure 4 and described herein below.

Only peptides APP 597-612, 597-624, 597-638, and N-dansyl-Ile-Ser-Glu-Val-Lys-Met-Asp-Ala-Glu-Phe-Arg-His-Asp-Asp-Asp-Asp (SEQ ID NO: 1) were able to inhibit C286.8A binding in a dose-dependent fashion. These peptides contain respectively amino acids 1-16, 1-28, 1-42 and 1-7 of the beta-amyloid sequence (numbering from the N-terminal aspartate residue). Peptides devoid of any beta-amyloid sequence such as APP 645-695, or containing the beta-amyloid peptide sequence 12-28 or 25-35 (APP 608-624 and APP 621-631 respectively) did not inhibit binding of the monoclonal antibody to the homologous antigen. These results show that the reactive epitope for this monoclonal antibody resides at least in part, in the first 7 amino acids of the A4 region of human APP, ie, APP 597-601.

### Example 7. Purification of recombinant holo-APP 695.

Initial studies of APP C-terminal processing were performed using recombinant APP695 expressed in CHO cells as described in Example 4, above, and purified as described by Method 1, below. Characterization experiments using this substrate are described in Example 8 below and led to the identification of six potentially different APP degrading enzymes capable of C-terminal processing.

Subsequently, a baculovirus expression system was developed (see Example 4), providing higher APP levels than could be achieved with the CHO expression system. The purification of holo-APP695 from the baculo virus system is described in method 2 below. The purified baculo virus derived holo-APP 695 was used to conduct the protease characterisation experiments described in Examples 9. and 10 below.

All steps were performed at 0 to 4°C unless indicated otherwise. Holo-APP 695 was detected by immunoblot analysis using an anti human APP 695 C-terminal antibody essentially as described in Example 8, below.

## Method 1. Purification of holo-APP695 from a Stably Transfected CHO Cell Line.

**a) Isolation of plasma membranes.** Whole cell pellets (179 g) from continuous culture of CHO cells in roller bottles (See Example 4) were collected by centrifugation (1500g X 5 min), and resuspended to a total volume of 600 ml in 50 mM tris-HC1 buffer pH 8.0 containing sodium chloride (30 mM), magnesium chloride (1 mM), EDTA (10 mM), PMSF (200 $\mu$g/ml), E-64 (42 $\mu$g/ml) and pepstatin (3.8 $\mu$g/ml). The cells were homogenized using a teflon potter (10 return strokes), then layered (25 ml per centrifuge tube) onto 10 ml of homogenization buffer containing 41% sucrose and devoid of the protease inhibitors EDTA, PMSF, E-64 and pepstatin. Following centrifugation (26,800 RPM X 60 min, in a Beckman SW-28 rotor, the interfacial layer was carefully removed (approximately 150 ml in combined volume), diluted with an equal volume of homogenization buffer (minus protease inhibitors), resuspended with a teflon potter (3 return strokes), and recentrifuged as described above to yield a tightly packed pellet. The supernatant was decanted and the pellet resuspended in 100 ml total volume with 50 mM tris HC1 pH 8.0 (teflon potter 3 return strokes). Recentrifugation (50,000 RPM X 60 min in a Beckman 70 Ti rotor), yielded a pellet which was resuspended to a total volume of 57 ml in 50 mM tris HC1, pH 8.0.

**b) Solubilization of Plasma Membranes.** Thirty seven milliliters of the above resuspended CHO plasma membrane preparation were added sequentially to a cocktail of protease inhibitors and stock 20% (v/v) triton X-100 to achieve the following component concentrations: EDTA (1 mM), E-64 (24 $\mu$g/ml), PMSF (53 $\mu$g/ml), pepstatin A (11 $\mu$g/ml), and triton X-100 (2.2% v/v, final), in the homogenization buffer (total solubilization volume of 45 ml) described above. After gently rocking of the mixture at 4°C for 30 min, the non-solubilized material was removed by centrifugation (50,000 RPM X 40 min in a Beckman 70 Ti rotor). The supernatant containing solubilized holo-APP was filtered through a 0.45 $\mu$M disc filter.

**c) Purification of solubilized holo-APP 695 by strong anion exchange chromatography.** The above supernatant containing holo-APP 695 was diluted with an equal volume of distilled water and applied to a Mono-Q RH 10/10 column previously equilibrated with 20 mM tris-HCl buffer pH 8.0 containing 0.1% triton X-100. Once loaded the column was eluted in a linear gradient of 0 to 1M NaC1 contained within a total volume of 210 ml of equilibration buffer. The flow rate was maintained at 3 ml/min throughout. Proteins eluting between a conductivity range of 17 to 22 mmho (4°C) contained the majority of immunoreactive APP 695, and were combined and dialyzed for 4 hours versus 2L of 5 mM tris-HC1 pH 8.0 containing 0,025% triton X-100, and clarified to remove slight turbidity by centrifugation (26,800 x 60 min in a Beckman SW 28 rotor).

**d) Heparin agarose chromatography.** The clarified sample was applied to a column of heparin agarose (15 x 1.6 cm) previously equilibrated with dialysis buffer. Upon loading a light brown band formed within the top 1/3 of the column. Once loaded, 5 min fractions were collected (a flow rate of 1 ml/min was used throughout). The column was then eluted stepwise with 85 ml of equilibration buffer in which the sodium chloride was successively adjusted to the following final concentrations: 0, 150, 300, 600, and 2000 mM. The majority of the immunodetectable holo-APP eluted at 600 mM NaCl, with the next quantitative fraction being recovered at 300 mM. The APP recovered at 300 mM and 600 mM NaC1 were collected separately and stored in aliquots at -80°C. The APP used in the following studies were from the 300 mM fraction. The yield of partially pure APP from the 300 mM heparin agarose eluent was 5.5 $\mu$g (Bradford assay) per gram of wet CHO cell pellet. The APP in the preparations was judged to be about 25% pure based upon SDS PAGE analysis.

## Method 2. Purification of Holo-APP695 from Recombinant Baculo Virus Infected Insect Cells.

a) <u>Solubilization of cell pellets.</u> The cell pellets harvested from two 5L fermentation runs were combined (total 8.9 g of detectable protein), added to 160 ml of 0.32 M sucrose containing the following inhibitor: pepstatin A (25 ug/ml); leupeptin (25 ug/ml); chymostatin (25 ug/ml); antipain (25 ug/ml); aprotinin (25 ug/ml), benzamidine (4 mg/ml), PMSF (0.87 mg/ml), and EDTA (25 mM), and homogenized by teflon potter (10 return strokes). The homogenate was centrifuged (105,000g X 1 h in a Beckman 70 Ti rotor) and the pellet was then resuspended by teflon potter (10 return strokes) in 160 ml of 10 mM Tris-HCl buffer pH 7.5 containing 0.5 M NaCl and the same inhibitors and concentrations as listed above. After brief sonication (Branson Sonifier Cell, 2 min power level 4), Triton X-100 was then added to a final concentration of 5 % (v/v), and the suspension was gently stirred for 20 min at 4 C. The mixture was centrifuged (50,000 RPM X 60 min, in a Beckman Ti 70 rotor), and the first supernatant (574 mg of protein) carefully removed for heparin-agarose chromatography. The pellet was resuspended by teflon potter (20 return strokes) in 160 ml of 10 mM tris-HCl buffer pH 7.5 containing 0.5 M NaCl, and each of the inhibitors at the concentrations listed above. Solubilization with 5% (v/v) triton X-100, and subsequent centrifugation was performed as described above to yield a second solubilized supernatant (683 mg of protein).

b) Radial flow chromatography on heparin-agarose. Both of the supernatants obtained above were purified separately on heparin agarose as follows. The supernatants were diluted by addition of purified water and 1M Tris pH 9.5 to a volume of 3.5 L, a conductance of 1.8 mmho, and a pH of 8.0, and applied to a Superflow 250 column (Sepragen) containing 250 ml of packed resin and previously equilibrated with 5 mM Tris-HCl buffer pH 8.0 containing 0.1 % triton X-100. Once loaded, the column was washed with 3L of equilibration buffer and then eluted with equilibration buffer containing 600 mM NaCl. A flow rate of 30 ml/min was used throughout. Fractions (45 ml were monitored for A 280 nm, total protein (Bradford assay), and the levels of immunoreactive APP deteced by immunoblot against the anti APP C-terminal antiserum of example 6 i). Fractions containing significant APP were combined and subject to antibody affinity chromatography.

c) Antibody affinity chromatography. The 600 mM elution pool from the purification of the first (containing 276 mg of protein) and second supernatant (containing 113 mg of protein) on heparin-agarose were combined, adjusted to pH 8.3, and applied to an antibody affinity column (10.5 X 1.5 cm) comprising affinity purified C-terminal antibody coupled to sepharose as described in example 6 ii), and previously equilibrated with 100 mM sodium bicarbonate buffer pH 8.3 containing 500 mM NaCl, 0.1 % triton X-100. Chromatography was performed at a flow rate of 1 ml/min throughout. Once loaded, the column was washed with 70 ml of equilibration buffer, and then eluted with 50 ml of 100 mM glycine, pH 2.4 containing 0.1% triton X-100. Fractions (5ml) were collected into 0.5 ml each of 1M tris-HCl pH 8.0, and monitored for A280 nm, total protein (Bradford assay), and the presence of immunodetectable APP as above. Fractions containing significant APP were combined. The combined heparin agarose eluent was cycled through the affinity pufication procedure a total of five times. The APP pool recovered from each successive purification was combined for a total of 9 mg of APP.

d) Strong anion exchange chromatography. Combined fractions from antibody affinity chromatography (9.0 mg of protein) were applied to a mono-Q HR 5/5 column previously equilibrated with 20 mM tris-HCl buffer pH 8.0 containing 0.025 % (v/v( triton X-100, and 150 mM NaCl. Once loaded, the column was eluted with a linear 0.15 to 1M NaCl gradient in a total of 70 ml. A flow rate of 0.5 ml/min was used throughout. Eluted fractions containing significant immunodetecable APP were combined and stored in aliqouts at -80 C until used. The final preparation of APP was >95% pure based on SDS-PAGE developed with coomassie brilliant blue stain, exhibited an amino acid composition that was within 86% agreement with the theoretical composition, and exhibited the following N-terminal sequence for the mature protein: Leu-Glu-Val-Pro-Thr-Asp-Gly-Asn-Gly-Leu-. 5.6 mg of purified protein was obtained from the pellet from the two 5 L fermentation runs.

Amino acid analysis was performed essentially as described elsewhere (Dupont, D.R., Keim, P.S., Chui, A.H., Bello, R., Bozzini, M., and Wilson, K.J., "A comprehensive approach to amino acid analysis", in Techniques in Protein Chemistry, ed. by Tony E. Hugli, Academic Press, 284-294 (1989)). Samples were hydrolyzed under argon in the vapor phase using 6N hydrochloric acid with 2.0% phenol at 160°C for 2 h. Phenylthiocarbamoyl-amino acid analysis was performed on an Applied Biosystems model 420A Derivitizer with on-line model 130A Separation System and Nelson Analytical model 2600 Chromatography Software.

**Example 8. The immunoblot assay for the detection of the degradation of APP 695 catalyzed by human brain protease subfractions.**

**a) Incubation with substrate APP**

i) 5 ul aliquots of ion-exchange fractions (obtained from steps as described in Example 1) or concentrated pools of fractions (Example 3) are incubated for 24 hrs at 37°C with recombinant human APP 695 (10.75 ul), which was adjusted to 140 mM final in MES buffer pH 6.5 by the addition of the required amount of 2M stock buffer. The final buffer concentration in the incubation was 95 mM, pH 6.5. During the incubation time, proteolytic degradation of some of the APP 695 occurs to yield lower Mr fragments.

ii) The proteolytic reaction was terminated by addition of 7.5 µl, of the following 3X Laemlie SDS-PAGE sample buffer: 1.5 M Tris HC1, pH 8.45, containing 36% (v/v) glycerol and 12% (v/v) SDS, 10% (v/v) 2-mercaptoethanol, and trace bromophenol blue tracking dye. Samples were heated (100°C X 8 min), and then cooled.

**b) SDS PAGE analysis:**

The reaction mixtures (15 µl) were applied to the wells of a 10 to 20% acrylamide gradient Tricine gel (routinely a 1.0 mm thick, 15 well Novex precast gel, Novex Experimental Technology, San Diego, CA). The gel was run under constant voltage conditions, and at 50 V until the sample enters the gel whereupon the voltage was raised to 100 V. Electrophoresis was discontinued when the tracking dye reaches to within 0.5 cm of the gel bottom. The gels were calibrated using prestained Mr markers ranging in Mr from 3 to 195 kDa (Bethesda Research Laboratories, Gaithersburg, MD.). Ten microlitres each of a kit containing high and low molecular weight markers were mixed with 10 μl of 3X sample buffer, and treated as described in section (a)(ii). The following molecular weight marker proteins were present in the kit as pre-stained markers: Myosin H-chain (196 kDa); phosphorylase B (106 kDa); bovine serum albumin (71 kDa); ovalbumin (45.3 kDa); carbonic anhydrase (29.1 kDa); betalactoglobulin (18.1 kDa); lysozyme (14.4 kDa); bovine trypsin inhibitor (5.8 kDa); and insulin A and B chains (3 kDa).

**c) Immunoblotting:**

i) The gel was then transferred to a mini trans-blot electrophoresis cell (Biorad Labs, Richmond, CA.). Proteins were electro-blotted onto a ProBlott (TM) membrane (Applied Biosystems, Foster City, CA.), for 1 hour at 100 V (constant), using the following transfer buffer maintained at 4°C:20 mM Tris HC1 buffer pH 8.5 containing 150 mM glycine and 20% (v/v) methanol.

ii) The ProBlott membrane was removed and placed in 15 ml of blocking buffer of the following composition for 1 hour at room temperature: 5% (w/v) non-fat dried milk in 10 mM Tris HC1 buffer pH 8.0 containing 150 mM NaCl.

**d) Immunodetection of APP and C-terminal degradation products:**

The membrane was transferred to 15 ml of blocking buffer containing a 1:1000 dilution of rabbit polyclonal antiserum elicited to a synthetic human APP 695 C-terminal peptide immunogen and incubated at 4°C over night.

The membrane was rinsed with three successive 15 ml volumes of blocking buffer with gentle shaking for 5 minutes. The membrane was then transferred to 15 ml of blocking buffer containing a 1:1000 dilution of alkaline phosphatase-coupled Goat anti-Rabbit IgG (Fisher Scientific, Pittsburgh, PA.), and incubated at room temperature for 90 minutes. The membrane was then rinsed with three successive 15 ml volumes of blocking buffer with gentle shaking for 10 minutes.

The membrane was next washed with three consecutive 15 ml volumes of alkaline phosphatase buffer for 5 minutes each, comprising: 100 mM Tris HC1 pH 9.5, containing 100 mM NaC1 and 5 mM $MgC1_2$. The gel was next incubated in the dark with 15 ml of 100 mM Tris HC1 pH 9.5, containing 100 mM NaC1, 5 mM $MgC1_2$ and 50 μl of BCIP substrate (50 mg/ml, Promega, Madison, WI.) and 99 μl of NBT substrate (50 mg/ml, Promega). Incubation was continued until there was no apparent further intensification of low Mr immunoreactive bands (typically 3 hours at room temperature). The gel was then rinsed with deionized water and dried.

**Analysis of the capacity of Mono-Q pools of subfractionated human AD cortex to enzymically degrade APP 695 to generate C-terminal fragments.**

Each of the P-2, S and M pools described in Example 3 were subject to the immunoblot assay described above. The specificity of the immunologic detection method, in combination with the use of the authentic APP substrate molecule provide a selective method to detect the activity of the APP degrading enzymes in comparatively crude biologic extracts, avoiding the need to use highly purified enzyme preparations. Thus, certain of the partially purified pools possessed a proteolytic activity which was capable of formation of C-terminal APP fragments in a time dependent manner. Representative examples of the immunoblot analysis of human AD brain are shown in Figure 2 for the P-2 V (panel a), M III (panel b) and S I (panel c), as well as for individual fractions prior to pooling of a P-2 VII pool (panel d).

Time course experiments, for example as depicted in Figure 2f, for pool M III showed that these fragments were not present in the substrate or enzyme fractions at time 0. Furthermore, incubation of the substrate alone did not result in their formation (for example see Figure 2a, lane 2, Figure 2d, lane 2, Figure 2f, lane 8). The size range of the bands varied between Mr approximately 11.5 kDa and 25 kDa, depending upon the enzyme fraction, but the number of different products formed in the reactions were surprisingly low. At pH 6.5, eight out of a total of 39 AD pools were found to have such activities. The pools could be distinguished from each other based upon i) brain sub-fraction, ii) ionic strength of column elution, and iii) qualitative APP cleavage pattern.

Six selected pools (designated "M-III, M-VIII, S-I, S-III, P-2 V, and P-2 VII") were found to contain significant APP degrading activity. Corresponding control brain pools also contained some of the above activities, but it was not possible to determine whether the levels of the activities were different or not, between control and AD pools. Each of the above six pools had an enzyme activity capable of forming an 11.5 kDa APP C-terminal fragment.

The proteolytic product of MR 11.5 kDa was of particular interest because in further studies it was usually the major immuno-detectable C-terminal product, and was found to co-migrate with a recombinant C-terminal fragment of APP comprising an open reading frame that would start with the N-terminal aspartate of the beta-amyloid peptide and extend to the C-terminus of the full length molecule (the C-100 fragment). This co-migration is exemplified in Figure 2d. The implication of this is that the 11.5 kDa band is the product of endoproteolysis of APP at or near the N-terminus of the A4 region, and that the above protease activities capable of forming this fragment might play a role in vivo, in the genesis of amyloidogenic peptides.

Figure 2d shows that at least in the case of P2 pool VII, the 11.5 kDa C-terminal enzymatic product of APP proteolysis is capable of aggregation. In addition to the appearance of the peptide band at Mr 11.5 kDa which comigrates with the PMTI 100 driven C-100 standard, and the 18 kDa fragment, there appear other bands at Mr 24.3, 27.4 and 35.5 kDa. The 24.3 and 35.5 kDa bands are of a Mr expected for dimers and trimers, respectively, of the C-100 fragment, and roughly comigrate wth the corresponding faint bands in the C-100 which are due to aggregation (see Example 5 for a details).

Figures 2a-2c also help to show that the assay can be used to examine the effect of classical protease inhibitors. For example, it is apparent from Figure 2a, that P-2 V is inhibited partially by methanol and completely by methanolic pepstatin A, while M-III (Figure 2b) and S-I (Figure 2c) are both completely inhibited by aprotinin and cystatin. Thus, the assay, in one embodiment, is applied to the search for novel in vitro inhibitors of the APP degrading enzymes. The potent compounds thereby identified are tested for in vivo efficay using a suitable animal model such as a transgenic animal designed to overexpress APP or a beta-amyloid-containing fragment thereof.

Table 4, below, summarizes some of the properties of the six main pools of APP degrading activity recovered from the Mono-Q fractions, including peptide product sizes, apparent pH dependence for product formation, and the effects of commercially available protease inhibitors.

### Table 4

**Properties of human AD brain fractions active in APP proteolysis[1].**

| Pool[2] | Conductance (mmho) | Fragment size[3] (kDa) | Optimum pH | Trial[4] | Inhibitors[5] (pH 6.5) |
|---|---|---|---|---|---|
| M III | 1.6 | 11.5 | (6.5) | A,B | aprotinin cystatin |
| | | >11.5 | (5.0) | – | N.D. |
| S I | 1.6 | 11.5 | (6.5) | A,B | aprotinin cystatin |
| | | >11.5 | (6.5-8.0) | A,B | cystatin |
| P2 V | 1.6 | 11.5 | (6.5) | A,B | pepstatin A aprotinin |
| | | 18.0 | (8.0) | – | N.D. |
| P2 VII | 2.5-5.2 | 11.5 | (6.5-8.0) | A | PMSF |
| | | >11.5 | (6.5-8.0) | A | PMSF |
| | | 18.0 | (6.5-8.0) | A,B | N.I. |
| S III | 5.0-6.8 | 11.5 | (8.0) | A | N.I. |
| | | >11.5 | (8.0) | A | N.I. |
| | | 18.0 | (8.0) | A,B | N.I. |
| M VIII | 15.0-16.0 | 11.5 | (8.0) | A,B | N.I. |
| | | >11.5 | (8.0) | A,B | N.I. |
| | | 18.0 | (8.0) | B | N.I. |

[1]Reactions were performed as described in Example 8. using pools prepared and concentrated according to Example 3

[2]Concentrated protease pools as defined in Example 3

[3]Specific C-terminal APP fragments (products) from proteolysis

[4]The inhibitors studied were:

**trial A:** PMSF (0.8mM), EDTA (7.7mM), Pepstatin A (400$\mu$M), E-64(260$\mu$M);

**trial B:** EGTA (1mM), cystatin (20$\mu$M), captopril (300$\mu$M), aprotinin (15$\mu$M), N-dansyl-Ile-Ser-Glu-Val-Lys-Met-Asp-Ala-Glu-Phe-Arg-His-Asp-Asp-Asp-Asp (SEQ ID NO: 1) (90$\mu$M).

[5]Compounds causing complete inhibition are listed.

N.D. = not determined due to low or inconsistent levels of activity; N.I. = no inhibition observed.

Some of the activities possessed pH optima in the alkaline range, and were unlikely to be due to the actions of lysozomal cathepsins. This observation is significant because several investigators have reported that pathologic APP processing is performed by proteases within the endosomal-lysozomal pathway (Cataldo et al., 1990, Proc. Natl. Acad. Sci USA 87:3861; Benowitz et al., 1989, Experimental Neurology 106:237; Cole et al., 1989, Neurochem Res 14:933). Enzymes within this pathway would be expected to exhibit acidic pH optima.

Based on the available data, M-III and S-I are highly similar by all the listed criteria, and probably represent the same enzyme cross contaminating each of the S and M fractions. It is probable, therefore, that human brain contains a minimum of five different protease activities capable of degrading APP to yield a 11.5 kDa C-100-like product fragment.

Table 4 shows that some of the activities were insensitive to inhibition by any of the inhibitors tested, and these enzymes may represent members of an unusual group. The activities involved in the formation of 11.5 kDa C-100 fragments in M-III and S I, are either of serine or cysteine type, or represent members of an unusual group. Alternatively, these fractions may contain both a serine and cysteine protease with both enzymes playing an obligatory (sequential) role in the production of C-100. P2 V contains both an aspartic protease activity and a serine protease activity. P2 VII contains a serine protease activity based upon its sensitivity toward PMSF. However, in subsequent studies pepstatin-inhibitable activity was also noted, indicating the co-localization in P2 of an aspartic protease along with the serine protease activity in Table 4. None of the enzyme activities in S III or M VIII were sensitive to any of the inhibitors tested. In no case was it possible to demonstrate inhibition of APP degradation by co-incubation of the enzyme pool with the N-dansyl peptide substrate used in Example 3.

Comparison of the recovery of APP degrading activities (Example 8) with the peptidase activities of the Mono-Q pools (Example 3 and Figure 1) clearly shows that there is little correlation between the two activities. Thus, the APP degrading activities were largely contained in pools that exhibited comparatively little peptidase activity. This suggests that the APP degrading activities are poor peptidases and may require an intact folded APP substrate for activity, or alternatively (but less likely) the peptides selected represent the wrong locus for pathologic APP processing. Regardless, this finding explains why other investigators have been unsucessfull in identifying common APP degrading enzymes using assays based on peptide substrates.

From the above considerations, it is concluded that the present assay is of a sufficient specificity to enable the isolation of specific APP degrading enzymes from human brain.

In further studies, we have used the immunoblot assay to track the recovery of the P-2 VII associated APPase. Work was focused on this pool because it represented the most abundant of the six characterized activities, and because it generated C-terminal fragments that seemed to be amyloidic (Figure 2d). It represents the major activity recoverable from ionexchange separation of the P-2 subfraction, and is eluted at a point in the gradient which did not coincide with the main peaks of peptidase activity.

The P-2 VII fractions displaying APPase were pooled and subject to size exclusion chromatography on two tandem Superose 12 columns (Pharmacia). Peptidase and APPase activities in the eluted fractions were analyzed (Figure 3a). While the K-M cleavage activity seemed to overlap in part, the peak of M-D activity once again did not coincide with the peak of APPase. Calibration of the chromatography against known molecular weight markers yielded a median Mr apparent of 31.6 kDa with an uncertainty of plus or minus 6.5 kDa for the APPase activity of the P-2 VII fractions (Figure 3b).

### Example 9. Identification of Cathepsin D as an APP C-terminal processing enzyme.

Having obtained greater quantities of holo-APP695 by using the baculovirus expression system described in Example 4, Method 2, and purification scheme of Example 7, Method 2, it became possible to track the recovery of APP degrading enzymes in individual column fractions from the purification of human brain enzymes, rather than assess the content of APP degrading enzymes in pools of fractions made on the basis of peptidase activity (as had been done in Example 8.

The content of APP degrading enzyme activity is shown in Figure 5 for individual mono-Q fractions from the purification of solubilized P-2 fraction according to the method of Example 1. When compared with a similar analysis of soluble and microsomal fractions subjected to Mono-Q chromatography, the relative staining intensity for enzymatic C-terminal APP fragments was consistently greatest in the P-2 subfraction from Mono-Q. APP degrading activity in the P-2 was recovered from Mono-Q as two distinct migration peaks (A and B, Figure 5).

Peak A eluted in the loading and low ionic strength wash, i.e. in a region roughly corresponding to the recovery of P-2 V, seen in our initial studies (Table 4), whereas peak B overlapped with the pooled region in which P-2 VII activity was previously observed (Table 4), and shown to comprise both serine and aspartic protease activities. Similar sized degradation products were observed with both the peak A and B activities at Mr approx. 28, 18 and 14 and <11 kDa, although the relative staining intensity of the 18 kDa band was much greater in peak B than in peak A. Peak B was pooled and subject to purification on superose 6HR as described in Example 1, Method 1. Eluted fractions contained two qualitatively distinct types of activity which overlapped in their elution profiles. The activity which produced an APP breakdown pattern most closely resembling that observed with the original peak B fractions (figure 5) was recovered in fractions 51 through 56 from gel filtration (figure 6 b and c), consistent with an apparent Mr of 15 to 25 kDa. This elution peak was preceded by elution of an activity which predominantly formed an 18 kDa breakdown product, and is presumably catalyzed by a protease of larger Mr apparent. This latter activity probably corresponds to the serine protease activity previously described in the P-2 VII pool in Example 8, Table 4. Active fractions from the gel-filtration purification of peak B (and within the 15-25 kDa Mr region) were tested for inhibition by clasical protease inhibitors (Figure 7). These studies confirmed that peak B activity was largely catalysed by an aspartic protease as determined by quantitative inhibition by Pepstatin A.

Comparatively few human aspartic protease are known. Those that have been identified include, Cathepsins D and E, Renin, and pepsin. To test the possibility that the activities that we observed might correspond to some of these enzymes, commercial preparations of human Renin (Calbiochem, Sandiego, CA catalog # 553864 ), and human cathepsin D (human liver, Cal Biochem, San Diego, catalog # 219401) were examined for their capacity to enzymically degrade baculo derived holo-APP.

The commercial preparation of cathepsin D used throughout the studies described herein was electrophoretically homogeneous on SDS-PAGE developed with silver stain, and exhibited an amino acid composition which showed 93 % agreement with the theoretical value based on the known protein sequence.

Whereas renin was innactive (not shown), cathepsin D selectively cleaved the APP so as to produce a similar pattern of C-terminal degradation products to those observed with P-2 peak B (Figure 5) described above from Mono-Q. Thus, commercial cathepsin D preparations degraded holo-APP in a time dependent fashion to produce major C-terminal products of approximate Mr 18 and 28 kDa. Inhibition of the activity by pepstatin A confirmed the involvement of cathepsin D in the reaction (Figure 8).

A commercial polyclonal antibody to human cathepsin D was obtained (Dako Corp, Carpinteria, CA, catalog # A561), and found to be reactive toward human cathepsin D on immunoblots, generating an immunoreactive band of Mr 28 kDa. The antibody was used in an immunoblot assay to examine if chromatography fractions from the mono-Q purification of either P-2, soluble or microsomal fractions contained immunoreactive cathepsin D.

Significant amounts of cathepsin D were observed in Mono-Q fractions of the P-2 and soluble fractions (data not shown) that possessed APP degrading activity. Interestingly, two chromatographically distinct peaks of cathepsin D reactivity were observed in the analysis of P-2 mono Q fractions each of which coincided with peaks A and B (not shown). The immunoreactive aspartic protease, cathepsin D associated with peak A activity coincided with the region in which P2 V of Example 8 had been previously identified. This suggested that peaks A and B could be due to multiple forms of cathepsin D. Multiple forms of cathepsin D have been described elsewhere and attributed to differences in post-translational modification of a single gene product. Immunoblot analysis of gel-filtration, column fractions from the further purification of P-2 peak B (Figure 5) showed the presence of a peak of cathepsin D immunoreactivity exactly co-incident with the peak of APP degrading activity (Figure 6B).

In addition to co-migrating with cathepsin D immunoreactivity and degrading APP similarily to cathepsin D, Peak B protease further purified by gel filtration exhibited the same pH optima (between pH 4-5) and ionic strength dependence as cathepsin D for formation of C-terminal APP degradation products (Figure 9). Finally, the immunoreactive band observed in the P-2 fraction exhibited a similar pI (4-6) to that reported for cathepsin D when subject to preparative IEF on Biorad Miniphor chromatography system (not shown). Collectively, these data strongly support the fact that the pepstatin sensitive APP protease activities observed following mono-Q fractionation of human brain P-2 are due to the action of cathepsin D.

The peptidase activity of the peak B protease (purified on gel filtration) and cathepsin D were then compared using the synthetic peptide N-Dansyl-Ile-Ser-Glu-Val-Lys-Met-Asp-Ala-Glu-Phe-Arg-NH2 (SEQ ID NO: 7) as the substrate. Both enzymes hydrolysed the peptide in a time dependent fashion albeit at quite low rates. For both enzymes, the major cleavage was observed at the -Glu-Val- bond and to a lesser extent at the -Met-Asp- bond (Figure 10). Note, that in Figure 10, most of the Met-Asp cleavage product was further converted to the Glu-Val product by the 24 hr. time point depicted. As expected, the peptidase reactions catalyzed by Cathepsin D and the P-2 enzyme preparation were both inhibited by pepstatin A.

Both enzymes exhibited acidic optima at pH 4 for the hydrolysis at the -Glu-Val- bond (Figure 11). Hydrolysis at the -Met-Asp- bond also exhibited an acidic optimum with cathepsin D (< pH 3.0), but with the P-2 enzyme, two optima were observed (at pH ,3.0 and pH 7.0), possibly due to participation of an additional contaminating P-2 protease in the reaction with a neutral pH optimum (Figure 11). Cathepsin D usually hydrolyses between hydophobic residues. However at acidic pH values, protonated (neutral) forms of the Asp and Glu side chains might appear sufficiently hydrophobic to satisfy the subsite binding requirements of the protease. The pKa of the Asp side chain is more acidic than the Glu residue, and would be protonated to a lesser degree than the Glu residue throughout the pH range examined in Figure 11. This may explain the lower cleavage rates at the -Met-Asp- bond with cathepsin D, and the hint at a lower pH optimum for cleavage (< pH 3) at this site when compared with the -Glu-Val-bond.

Further experiments explored the identity of the peptide bonds in APP that are cleaved by Cathepsin D. Larger amounts of APP were subject to cathepsin D hydrolysis at pH 5.0. Limited proteolysis under non-denaturing conditions was employed. Incubation mixtures were analysed by SDS-PAGE, immunoblotted, and then the individual product bands located either by coomassie staining or by immunodetection with the anti-beta-amyloid monoclonal described in Example 6(iii). The main bands located with coomassie blue were subject to N-terminal sequencing.

Figure 12 shows both a coomassie stained blot as well as an immunoblot (using the anti-beta-amyloid monoclonal antibody) of such a reaction mixture. As a control, incubations were also performed in the absence of cathepsin D (wherein cathepsin D would be added back to the incubation mixture after addition of SDS-PAGE sample buffer), or in the absence of APP 695 substrate. Eight main product bands were observed by coomassie staining (Figure 12a) of the complete incubation mixture, and which were also absent from either of the controls. Some but not all of those bands also reacted with the A4 monoclonal (Figure 12c), which recognises an epitope within the first 5 residues of the beta-amyloid peptide. N-terminal analysis of the coomassie stained products yielded the sequences listed in the table below.

## Table 5.

**N-terminal sequences of major proteolytic products following incubation of purified cathepsin D with holo-APP 695.**

| Proteolytic band # (Fig. 12a) | product size (kDa) | N$^a$-terminal sequence | peptide bond hydrolyzed |
|---|---|---|---|
| 1 | 3.9 | R-V-I-Y-E-R-M-<br>Q-A-V-P-P-R-P- | -L-R-<br>-L-Q- |
| 2 | 4.4 | Q-A-V-P-P-R-P-<br>R-V-I-Y-E-R-M- | -L-E-<br>-L-R- |
| 3 | 5.6 | V-K-M-D-A-E-F-<br>Q-A-V-P-P-R-P- | -E-V-<br>-L-E- |
| 4 | 6.3 | V-S-D-A-L-L-V- | -F-V- |
| 5 | 10.0 | V-S-D-A-L-L-V-<br>L-E-V-P-T-D-G-<br>V-K-M-D-A-E-F- | -F-V-<br>-A-L-<br>-E-V- |
| 6 | 15.8 | G-A-D-S-V-P-A- | -F-G- |
| 7 | 24.5 | L-E-V-P-T-D-G | -A-L- |
| 8 | 56.2 | L-E-V-P-T-D-G | -A-L- |

ᵃ The amino acid sequences were determined with an Applied Biosystems model 477A Protein Sequencer operated in the gas phase with on-line model 120A Analyzer and Nelson Analytical model 2600 Chromatography Software.

Where:   A = Ala, D = Asp, E = Glu, F = Phe, G = Gly, I = Ile, K = Lys, L = Leu, M = Met, P = Pro, Q = Gln, R = Arg, S = Ser, T = Thr, V = Val  and Y = Tyr.

With exception of band 7 all sequences were assigned for the first ten cycles. For band 7, sequencing was discontinued after cycle 6.

---

As expected several products were observed corresponding to cleavages that were largely consistent with those reported for cathepsin D hydrolysis of other substrates (Moriyama et al., 1980, J. Biochem, 88:619). Exceptions to the reported cathepsin D specificity included the -Glu-Val- cleavage to form the major product of band 3, and the minor product of band 5, as well as the -Leu-Arg- cleavage products of bands 1 and 2 (Table 5).

The cleavage of the -Glu-Val- bond at APP 593-594 is consistent with the observed capacity of the cathepsin D to cleave the corresponding bond in the peptide substrate (as described above) in a pepstatin inhibitable reaction. Cathepsin D usually hydrolyses between pairs of certain hydrophobic residues. Cleavage at the Glu-Val bond, though unexpected, probably occurs under acidic (pH 5) conditions due to protonation of the side chain of the glutamate residue (pKa = 4.25), rendering it neutral.

Indeed, it can be calculated that 18% of the -Glu- side chains should be protonated at pH 5.0. Such acidic conditions occur in lysozomes and secretory granules, or could be induced upon tissue damage, or following hypoxia or local ischaemia.

Most significantly, cathepsin D generated a 5.6 kDa product (band 3, Table 5), by atypical hydrolysis at the -Glu-Val- bond three amino acid residues N-terminal to the purported N-terminal -Asp- residue of the common form of beta-amyloid. The fragment was absent in the equivalent sections of the blot taken from the incubation without cathepsin D. Furthermore, the fragment is of the right size (5.6 kDa) to contain full length beta-amyloid peptide, and its generation suggests that cathepsin D must also cleave the APP at a second site close to the C-terminal region of the beta-amyloid peptide.

In fact, a precursor substrate for such a C-terminal cleavage was also identified in band 5, which exhibited an Mr (10.0 kDa). The size of this fragment suggests that it contains most if not all of the C-terminal domain and that it arose by a single -Glu-Val-cleavage at APP 593-594.

APP 695 contains numerous other peptide bonds that would seem to have been ideal substrates for cathepsin D cleavage yet were not cleaved by cathepsin D. The fact that they were not hydrolyzed reflects the high degree of sequestration of these sites away from acess to cathepsin D within the folded APP structure: most of the hydrophobic pairs would be expected to locate to the hydrophobic APP protein core. The same considerations explain why the sites that were shown to be hyrolysed by cathepsin D (Table 5) did not not always contain the optimal cathepsin D recognition motif. To be located on the protein surface, such sites would have to contain a greater degree of polarity or charge than would be ideal for cathepsin D catalysed cleavage. It is noteworthy in this regard that three of the five internal cleavage sites contained two proline residues each within eight residues of the scissile bond. Such residues are often associated with a brake in secondary structure or with turns which often are found at the protein surface.

In a parallel immunoblot (Figure 12c) several of the product peptides (located with arrows), reacted with the monoclonal antibody C286.8A to the N-terminal residues of beta-amyloid. These included a band at Mr 5.6 which migrated in the same position as band 3 in Figure 12a (Table 5) a doublet between Mr 9 to 10 kDa comigrating with band 5 in Figure 12a (Table 5), and a doublet at Mr 14 kDa, a doublet at 16 to 18 kDa comigrating with band 6, Figure 12a, and a band at Mr 40 kDa. Of the bands sequenced (Table 5), only bands 3, 5 and 6 comigrated with bands detected by immunoblot in Figure 12c. Consistent with this, only these same three bands in Table 5 were of the appropriate N-terminal sequence and size to contain the beta-amyloid epitope.

The time course of formation of the beta-amyloid immunoreactive degradation products described in Figure 12 was performed under slightly different molar ratios of cathepsin D and APP (Figure 13), both in the absence and presence of pepstatin A. In the absence of inhibitor, a time dependent accumulation of low molecular weight fragments was observed, starting initially with the formation of a bands at Mr approx. 16-18 and 28 kDa respectively. At 2hr, a band at Mr approximately 40 kDa was observed. While the 16-18 and 40 kDa bands further intensified beyond 2 hr., the intensity of the 28 kDa band remained constant beyond this time point. The intensities of the 16-18 and 40 kDa did not increase further beyond 8 hr. Between 8 hr. and 21 hr. there was a substantial increase in the intensities of detectable bands at Mr approx. 14, 10 and 5.6 kDa. Since these latter three bands did not intensify in parallel with either the 16-18, or 40 kDa, it is probable that the 14, 10 and 5.6 kDa bands were derived from secondary degradation of either or all of the 16-18 or 40 kDa bands. The 16-18, 10 and 5.6 kDa bands described in Figure 13 correspond to the same Mr bands listed in Table 5 and shown in Figure 12c. All of the bands observed in Figure 13 were inhibited by pepstatin A confirming that they arose by the action of cathepsin D.

The implication of cathepsin D as a major protease in amyloidosis of Alzheimer's Disease now explains other observations made concerning the disease. Firstly, there is growing evidence that APP accumulates in lysozomes, and is processed there to yield amyloid bearing fragments (Haas et al., 1992, Nature 357:500). Amyloid deposition is favored at the acid pH of the lysosome (Burdick et al., 1992, J. Biol. Chem. 267:546). Secondly, while cathepsin D is a lysozomal protease, it has also been shown by histochemistry to be present in significant levels associated with amyloid deposits in Alzheimer's brain (Cataldo et al., 1990, Proc. Natl. Acad. Sci USA 87:3861).

Thirdly, beta-amyloid released by cells in culture comprises a minor N-terminal sequence starting at residue Val 594 (Haas et al., 1992, Nature 359:322) which is three amino acids N-terminal to the more abundant sequence beginning at the Asp 597 residue commonly seem in beta-amyloid 1-42. The minor sequence probably arises by direct endoproteolysis at the -Glu-Val- bond at position 593-594, ie the same site as shown presently to undergoe specific proteolysis by cathepsin D. It is emphasised here that since cathepsin D can hydrolyse both the -Glu-Val- and -Met-Asp- bonds, it has the necessary specificty to form both of the beta-amyloid fragments sequenced by Haas et al.

Fourthly, the cysteine preoteases inhibitors E-64 and leupeptin were without effect on the release of beta-amyloid by LC-99 cells while general lysozomal inhibitors blocked the release (Shoji et al., 1992, Science, 258:126), showing that beta-amyloid formation by these cells was catalysed by a lysozomal enzyme other than a cysteine protease. A remaining candidate protease for such a reaction would be lysozomal cathepsin D which is not inhibited by the cysteine protease inhibitors used in their studies.

Finally, APP contains a stretch of hydrophobic residues between the C-terminus of beta-amyloid and the membrane anchore sequence. Some of the peptide bonds in this region could be hydrolysed by cathespin D. Indeed the -Leu-Val- peptide bond at position 645-646 is highlighted by the PEPTIDESORT computer program as being a probable cathepsin D recognition site. This site is close to the position of three of the point mutations shown to co-segregate with certain forms of Familial Alzheimer's Disease (FAD). Cleavage within this region as well as the -Glu-Val- bond at positions 593-594 could account for the size of band 3 in Table 5. The FAD mutations at this site could augment the rates of APP cleavage within this region by cathepsin D.

Finally, a double mutation of APP from -Lys-Met- to -Asn-Leu-at positions 595-596 also cosegregates with familial AD. This mutation makes it unlikely that a specific amyloidogenic protease exists with specificity for cleavage about the -Lys-Met- bond, since the mutation would be expected to abrogate rather than augment cleavage by such an enzyme. Rather, the likelyhood is increased that the primary endoproteolysis yielding beta-amyloid occurs adjacent to and preferably N-terminal to this dipeptide. The -Glu-Val- bond represents the closest N-terminal site left unaffected in the S1 and S1' positions.

Conceivably, the -Asn-Leu- mutation at S2'and S3' sites to the -Glu-Val- scissile bond could augment cleavage by cathepsin D. To test whether this was the case, we compared the capacity of both cathepsin D and the P-2 enzyme peak B to hydrolyze the Substrate N-dansyl-Ile-Ser-Glu-Val-Lys-Met-Asp-Ala-Glu-Phe-Arg-NH2 (SEQ ID NO: 7), and a similar peptide in which the K-M pair was replace with NL thereby mimicking the above described FAD mutation (Figure 14). While both enzymes cleaved the wild type peptide at the M-D and EV bonds in longer time frames, very little cleavage was observed in the short incubation time shown in Figure 14. By contrast, cleavage of the mutant peptide by both enzymes occurred with initial velocities between 30 to 50 times faster than observed with the wild type peptide. The single metabolite thereby generated exhibited a retention time of 4.4 min, and had not been seen previously using the wild type peptide. This unidentified product must therefore result from hydrolysis either between the N-L pair or C-terminal to the L residue. In either case, this result is consistent with the notion that the -NL- mutation observed in this particular early onset FAD causes enhanced rates of beta-amyloid formation by providing a site that is more rapidly cleaved by the amyloidogenic protease cathepsin D. The increased rates of beta-amyloid accumulation that could result, could trigger the early onset form of Alzheimers Disease linked to this APP mutation.

The identification of cathepsin D as a serious candidate for the primary amyloidogenic protease of Alzheimer's Disease, significantly aids the effort of development of therapeutic inhibitors for the disease. For example specific cathepsin D inhibitors could provide therapeutic benefit by inhibiting the toxic accumulation of beta-amyloid. The new information provided herein makes the comparatively straightforward to rationally design tight-binding inhibitors as has been accomplished for the design of novel inhibitors of other aspartic proteases such as renin and HIV-protease.

Alternatively cathepsin D can now be adapted for use in a high throughput screen using an in vitro peptidase assay so as to identify therapeutic inhibitors through random or semi-random search of chemical libraries. A suitable assay for such purposes could include the N-Dansyl-peptide assay described in Examples 2 and 3 of the present invention.

**Example 10. Identification of a serine protease with specificity for C-terminal APP processing.**

Table 4, showed that human brain contains serine proteases capable of C-terminal processing of recombinant APP, and that it some cases these serine proteases were inhibitable with aprotinin. To attempt a more facile isolation of such proteases, an alternate isolation scheme was devised (Example 1, Method 2) incorporating affinity purification on aprotinin-sepharose as an early step.

Application of this procedure for the further purification of the P-2 fraction was successful in the isolation of APP degrading activity (Figure 15). The active fractions recovered from the aprotinin-sepharose column by acid elution were further purified on a mono-Q column (Figure 16). Active fractions (Figure 16a) exhibited the capacity to form APP C-terminal fragments of 11 kDa, 14 kDa and 18 kDa, when analysed by immunoblot with a polyclonal antibody to the APP C-terminus (Figure 16b). The smallest products co-migrated with the recombinant C-100 standard. Reassay of APP degradation in the active fractions using an anti-beta-amyloid monoclonal antibody C286.8A led to the detection of the same three product bands (Figure 16b). Since the antibody C286.8A recognizes the first seven amino acid residues of the beta-amyloid peptide, as in Example 6(iii), this experiment shows that all three products contained full length beta-amyloid.

One or more of these product peptides could be amyloidic or give rise to beta-amyloid by further processing of these peptides C-terminal to the beta-amyloid region. The serine protease activity invloved in formation of these products could therefore play a role in amyloidosis.

The enzymic activities which formed the 11, 14 and 18 kDa product bands described above eluted as a broad peak from mono-Q and could perhaps have resulted from the action of more than one protease. Based on the recovery of A280 nm absorbing components from the mono-Q column, three different pools of proteolytic activity were prepared from the mono-Q column fractions termed pool X, Y and Z (Method 2, Example 1).

Enzymatic activity was recovered in the void volume during chromatography of each pool on superdex 75 (data not shown), consistent with an apparent Mr >75 kDa, although possible protein aggregation during chromatography cannot be ruled out. Pool Y represented the purest pool when analysed on SDS-PAGE, and exhibited a major stained band at Mr of approx 100 kDa. Pool Y was selected for further characterization. The pH dependence for APP hydrolysis by pool Y showed an optimum between pH 7 and 9 (Figure 17a), and the enzyme activity was gradually inhibited by increases in sodium chloride concentration beyond 42 mM (Figure 17b). Studies of the inhibitor sensitivity of the enzyme (Figure 18a), confirmed that it was a serine protease, being inhibited by PMSF and aprotinin but unaffected by pepstatin A, E-64 or EDTA (Figure 18a). The serine protease inhibitor benzamidine was without effect on the enzyme, suggesting that it was unlikely to be a trypsin-like endoprotease. More likely the enzyme is of the chymotryptic family with specificity for cleavage of substrates containing a neutral hydrophobic residue at the S1 subsite.

Accordingly, further inhibitor studies (Figure 18b) showed that the activity of the pool Y protease was strongly inhibited by chymotrypsin inhibitor II, alpha-2-antiplasmin and TPCK. Weak inhibition was also observed with chymostatin and alpha-1-antichymotrypsin, but TLCK, did not inhibit at all. Cathepsin G has been suggested by others to play a role in APP processing, however immunoblot analysis of the pool Y protease fraction using a polyclonal antibody to cathepsin G failed to detect the presence of this serine protease. N-terminal sequencing of the 11, 14 and 18 kDa will identify the cleavage sites and is already ongoing.

**Example 11. Design of therapeutic cathepsin D inhibitors.**

This example utilizes the nomenclature of Schechter et al., 1967, Biochem Biophys Res Comm. 27:157, to describe peptide specificity, wherein the amino acids in the substrate which flank the scissile bond are numbered according to their position relative to the peptide bond being cleaved by the enzyme. Peptide substrate amino acid side chains N-terminal to the scissile bond are numbered consecutively as P1 to Pn with increasing distance from the scissile bond. Peptide substrate amino acid side chains C-terminal to the scissile bond are numbered consecutively as P1' to Pn'. The P1 and P1' amino acid side chains correspond to the amino acids involved in formation of the peptide bond which is to be cleaved. The side chains P1 to Pn and P1' to Pn' are envisioned to form specific interactions with a corresponding series of enzyme subsites S1 to Sn and S1' to Sn respectively. The interactions between the P side chains and corresponding S subsites contribute to the binding energy for stabilization of the protease-substrate complex, and thus confer specificity to the interaction. The approach taken to the development of peptidomimetic inhibitors could utilize either the N-dansyl peptide substrate assays of Examples 2 and 3, or the assay of holo-APP degradation described in Example 8, to make enzymologic measurements, in conjunction with purified cathepsin D.

**Quantification of optimal peptide length and sequence for proteolytic cleavage by cathepsin D in vitro.** Starting with a dodecapeptide peptide of sequence Dns-Ile-Ser-Glu-Val-Lys-Met-Asp-Ala-Glu-Phe-Arg-NH2 (SEQ ID NO: 7), the effect of shortening of the peptide either from the N-terminus or the C-terminus on the apparent kinetic parameters for enzymic hydrolysis would be determined at acidic pH and optimal ionic strength. The effect on Km and Vmax for hydrolysis of variation in the amino acids at each position in the peptide of optimal length would be determined.

**Inhibitor synthesis.** Peptidomimetic compounds would be synthesized containing essential amino acid sequences necessary for optimal cleavage (from 1 a,b above), and the appropriate spacer. The amino acid sequences in these peptides could be the same as those observed around the cleavage site in the APP substrate, eg. Glu-Ile-Ser-Glu-Val-Lys-Met-Asp (SEQ ID NO: 4) and Trp-His-Ser-Phe-Gly-Ala-Asp-Ser (SEQ ID NO: 5) or alternatively selected from those sequences found to confer optimal binding to cathepsin D based on studies of their potency for in vitro inhibition of cathepsin D. In the case of Glu-Ile-Ser-Glu-Val-Lys-Met-Asp (SEQ ID NO: 4) and Trp-His-Ser-Phe-Gly-Ala-Asp-Ser (SEQ ID NO: 5) the P1-P1' bond is E-V, and F-G respectively. Peptidic inhibitors would be synthesized that contain either the above sequences or sequences exhibiting optimal cathepsin D inhibition (including shorter variants perhaps containing N- and/or C- substitutions), in which the -CO-NH- atoms of the peptide bond between P1 and P1' are replaced with any of the following standard spacer groups and using appropriate synthetic routes so as to obtain any possible stereo-chemical configuration thereof: reduced amide, hydroxy isostere, ketone isostere, dihydroxy isostere, statine analogs, phosphonates or phosphoamides, reversed amides. Most of these inhibitors would function as transition state analogs. The potency of these first generation compounds as determined using either of the in vitro assays of the present invention (N-Dansyl-peptide assay of holo-APP degradation assay) could be optimized by any or all of the following:

i) Addition or deletion of flanking amino acid residues;
ii) Alteration of the type of amino acid side chain (D or L) at each position in the inhibitor;
iii) N- and C-terminal substitution with blocking groups such as boc or acetyl (N-terminally), or O-Me , O-benzyl, N-benzyl (C-terminally).

Beside the inhibitors rationally developed according to the above methods, other known cathepsin D inhibitors either in whole or in part could be used as therapeutic inhibitors for Alzheimers Disease, or as starting points for optimization of inhibitory potency and the development of new derivatives for therapy of Alzheimer's Disease. Such inhibitors include: 1-Deoxynojirimicin (Lemansky et al., 1984, J. Biol Chem. 259:10129); Diazoacetyl-norleucine methyl ester (Keilova at al., 1970, Febs Lett, 9:348); Gly-Glu-Gly-Phe-Leu-Gly-Asp-Phe-Leu (SEQ ID NO: 6) (Gubenseck et al., 1976, Febs Lett, 71:42); Pepsin inhibitor from Ascaris (Keilova et al., 1972, Biochem Biophys Acta. 284:461); pepstatin (Yamamoto et al., 1978, European Journal of Biochemistry 92:499).

## Claims

1.   A method for regulating formation of beta-amyloid protein with an inhibitor of at least one protease specific for the Precursor to the Alzheimer's Disease beta-amyloid protein.

2. The method of claim 1, wherein said inhibitor is selected from the group of inhibitors consisting of those specific for aspartic proteases and serine proteases.

3. The method of claim 2, wherein said inhibitor of aspartic proteases specifically inhibits cathepsin D.

4. The method of claim 2, wherein said inhibitor of serine proteases specifically inhibits a serine protease which is inhibited by alpha-2-antiplasmin, chymotrypsin inhibitor II, or TPCK, and which forms 11, 14 and 18 kDa APP C-terminal fragments at pH 7-9.

5. The method of claim 3, wherein said inhibitor is selected from the group consisting of 1-Deoxynojirimicin, Diazoacetyl-norleucine methyl ester, Gly-Glu-Gly-Phe-Leu-Gly-Asp-Phe-Leu (SEQ ID NO: 6), Ascaris Pepsin Inhibitor, and Pepstatin.

6. A method for preventing the formation of amyloid plaques in Alzheimer's Disease, comprising administering a therapeutic amount of an inhibitor to cathepsin D.

7. The method of claim 6, wherein said inhibitor is a transition state analog containing a reactive spacer selected from the group consisting of reduced amides, hydroxy isosteres, ketone isosteres, dihydroxy isosteres, statine analogs, phosphonates, phosphoamides, and reversed amides.

8. A method for identifying inhibitors of cathepsin D, comprising:
   a) incubating cathepsin D with a peptide substrate capable of being cleaved by cathepsin D to form a first incubate conducted under conditions at which the cathespsin D is catalytically active;
   b) incubating cathepsin D with a peptide substrate capable of being cleaved by cathepsin D in the presence of a potential inhibitor to form a second incubate;
   c) analyzing the amount of peptide products formed over a time period to calculate the product formation rate in said first and said second incubates; and
   c) calculating the reduced enzyme activity observed in the presence of the potential inhibitor, said reduction indicating inhibitory activity.

9. The method of claim 8, wherein said cathespsin D is human cathepsin D.

10. The method of claim 8, wherein said peptide substrate is N-dansylated.

11. A method for measuring the proteolytic activity of molecules capable of degrading amyloid precursor protein, comprising:
   a) incubating a physiological sample obtained from a human, in the presence of amyloid precursor protein substrate under conditions in which amyloid precursor protein degrading proteases in said sample are catalytically active;
   b) forming a gel with a portion of terminated incubation mixture of step (a);
   c) electrophoresing the gel of step (b) to obtain an electrophoretic migratory pattern representing separate polypeptide constituents;
   d) blotting said constituents of step (c) onto a membrane;
   e) contacting said blotted membrane from step (d) with anti-amyloid precursor protein antibody;
   f) reacting said blotted membrane with a second antibody that recognizes said anti-amyloid precursor protein antibody, said second antibody being coupled to a detectable ligand; and
   g) examining the intensity of staining of the blots in regions corresponding to fragments of a size sufficient to contain the beta-amyloid peptide sequence.

12. The method of claim 11 wherein said physiological sample is selected from the group consisting of brain tissue and cerebro-spinal fluid.

13. The method of claim 12 wherein said physiological sample contains cathepsin D.

14. The method of claim 11 further comprising treating said physiological sample to obtain a crude homogenate, a soluble fraction, or a detergent solubilized membrane fraction.

15. The method of claim 11 wherein said amyloid precursor protein substrate is translated from gene sequences containing point mutations.

16. The method of claim 11 wherein said amyloid precursor protein substrate corresponds to a C-terminal portion of the amyloid precursor protein.

17. The method of claim 11 wherein said anti-amyloid precursor protein antibody recognizes peptides selected from the group consisting of beta-amyloid peptides and C-terminal fragments of amyloid precursor protein.

18. The method of claim 17 wherein said C-terminal fragments comprise C-100 or beta-amyloid peptide fragments as detected by co-migration with recombinant C-100 or beta-amyloid size markers.

19. A method for identifying inhibitors of proteases specific for amyloid precursor protein, comprising:
a) forming a first incubate with a portion of a physiological sample obtained from a human and a selected amyloid precursor protein substrate, and
forming a second incubate with a second portion of said physiological sample obtained from a human, said selected amyloid precursor protein substrate and a test inhibitor;
b) terminating the incubations in step (a) after a predetermined duration;
c) forming gels with portions of the terminated reaction mixtures of step (b);
d) electrophoresing the gels of step (c) to obtain electrophoretic migratory patterns representing separate polypeptide constituents;
e) blotting said constituents of step (d) onto membranes;
f) contacting said membranes from step (e) with an anti-amyloid precursor protein antibody;
g) reacting said blotted membrane with a second antibody that recognizes said anti-amyloid precursor protein antibody, said second antibody being coupled to a detectable marker;
h) examining the intensity of staining of the blots in regions corresponding to fragments of a size sufficient to contain the beta-amyloid peptide sequence; and
i) comparing the intensities of the bands of the same size observed from said first and said second incubates.

20. The method of claim 19 wherein said proteases specific for amyloid precursor protein is cathepsin D.

FIG. 1a

FIG. 1b

FIG. 1c

FIG. 1d

FIG. 1e

FIG. 1f

44

1 2 3 4 5 6

FIG. 2a

1 2 3 4 5 6 7 8

FIG. 2b

1 2 3 4 5 6 7

FIG. 2c

38      39  40  41  42  43

+ Mr - + - + - + - + - + - APP ONLY  C-100

FIG. 2d

5 4 3 2 1

← C-100

FIG. 2e

8 7 6 5 4 3 2 1

FIG. 2f

47

FIG. 3a

FIG. 3b

# FIG. 4

BETA−AMYLOID

FRAGMENT #

| | |
|---|---|
| ●— | 1−28 |
| ▲— | 1−42 |
| ○— | 1−16 |
| △— | 1−7 |
| □— | 25−35 |
| ✕— | 12−28 |

APP FRAGMENT #

| | |
|---|---|
| ■— | 645−695 |

# FIG. 5A

PEAK A

# FIG. 5B

## FIG. 5C

Mr 33 34 35 C-100 36 37 38 APP-Ohrs

-APP

PEAK B

## FIG. 5D

Mr 39 40 41 C-100 42 43 44 APP-24hrs

-APP

FIG. 6A

# FIG. 6B

# FIG. 6C

FIG. 6D

FIG. 6E

FIG. 7

# FIG. 8

FIG. 9A

FIG. 9B

FIG. 10A

FIG. 10B

59

FIG. 10C

FIG. 10D

FIG. 10E

FIG. 10F

FIG. 11A

FIG. 11B

FIG. 12a

FIG. 12b

FIG. 12c

Lane # 11 12 13 14 15 16 17 18

Lane # 1 2 3 4 5 6 7 8 9 10

APP

APP

C100  + - + - Mr

C100  + - + - + - + - Mr

21 Hrs   8   APP-21hrs   APP-0hrs

4 Hrs   2   1   0

FIG. 13

FIG. 14A

FIG. 14B

FIG. 14C

FIG. 14D

EP 0 569 777 A2

I ml / min

4 min FRACTIONS

COLUMN, 1·5 x 10 cm

[ = 0·1 OD 280 nm

LOAD    WASH    ELUTE

0 5 10 15 FRACTIONS

←—20 mM tris HCl pH 7·0 ——→ ◄50 mM Na OAc /

500 mM NaCl pH 5·0

# FIG. 15a

# FIG. 15B-1

-APP

# FIG. 15B-2

APP

FIG. 16a

FIG. 16B

FIG. 16C

## FIG. 17A

lane #  1 2 3 4 5 6 7 8 9 10 11 12

## FIG. 17B

lane #  1  2 3 4 5 6 7 8 9 10 11 12 13 14 15

FIG. 18A

FIG. 18B

**SEQUENCE LISTING**

(1) GENERAL INFORMATION:

    (i) APPLICANT:    Paul P. Tamburini and Robert N. Dreyer

    (ii) TITLE OF INVENTION:    Methods for Detecting Beta Amyloid Precursor Protein Processing Enzyme

    (iii) NUMBER OF SEQUENCES: 8

    (iv) CORRESPONDENCE ADDRESS:

        (A) ADDRESSEE: Miles Inc.
        (B) STREET: 400 Morgan Lane
        (C) CITY: West Haven
        (D) STATE: Connecticut
        (E) COUNTRY: USA
        (F) ZIP: 06516

    (v) COMPUTER READABLE FORM:

        (A) MEDIUM TYPE: Floppy diskette
        (B) COMPUTER: IBM PC
        (C) OPERATING SYSTEM: MS-DOS
        (D) SOFTWARE: Word Perfect 5.1

    (vi) CURRENT APPLICATION DATA:

        (A) APPLICATION NUMBER:
        (B) FILING DATE:
        (C) CLASSIFICATION:

    (vii) ATTORNEY/AGENT INFORMATION:

        (A) NAME: Jae H. Kim, Esq.
        (B) REGISTRATION NUMBER: 32,907
        (C) REFERENCE/DOCKET NUMBER: MTI 224

(viii)   TELECOMMUNICATION INFORMATION:

        (A)   TELEPHONE:   (203) 937-2340
        (B)   TELEFAX:   (203) 937-2795

(2)  INFORMATION FOR SEQ ID NO: 1

    (i)  SEQUENCE CHARACTERISTICS:

        (A)  LENGTH: 16 amino acids
        (B)  TYPE: amino acid
        (C)  TOPOLOGY: linear

    (ii)  SEQUENCE DESCRIPTION: SEQ ID NO:1


        Ile Ser Glu Val Lys Met Asp Ala Glu Phe
         1               5                   10

        Arg His Asp Asp Asp Asp
                        15


(3)  INFORMATION FOR SEQ ID NO: 2

    (i)  SEQUENCE CHARACTERISTICS:

        (A)  LENGTH: 12 amino acids
        (B)  TYPE: amino acid
        (C)  TOPOLOGY: linear

    (ii)  SEQUENCE DESCRIPTION: SEQ ID NO: 2


        Ile Ser Glu Val Lys Met Asp Ala Glu Phe
         1               5                   10

        Arg His

(4) INFORMATION FOR SEQ ID NO: 3

    (i) SEQUENCE CHARACTERISTICS:

        (A) LENGTH: 11 amino acids
        (B) TYPE: amino acid
        (C) TOPOLOGY: linear

    (ii) SEQUENCE DESCRIPTION: SEQ ID NO: 3

      Ile Ser Glu Val Asn Leu Asp Ala Glu Phe Arg
       1              5             10

(5) INFORMATION FOR SEQ ID NO: 4

    (i) SEQUENCE CHARACTERISTICS:

        (A) LENGTH: 8 amino acids
        (B) TYPE: amino acid
        (C) TOPOLOGY: linear

    (ii) SEQUENCE DESCRIPTION: SEQ ID NO: 4

      Glu Ile Ser Glu Val Lys Met Asp
       1              5

(6) INFORMATION FOR SEQ ID NO: 5

    (i) SEQUENCE CHARACTERISTICS:

        (A) LENGTH: 8 amino acids
        (B) TYPE: amino acid
        (C) TOPOLOGY: linear

(ii) SEQUENCE DESCRIPTION: SEQ ID NO: 5


Trp His Ser Phe Gly Ala Asp Ser
1                    5



(7) INFORMATION FOR SEQ ID NO: 6


(i) SEQUENCE CHARACTERISTICS:


(A) LENGTH: 9 amino acids
(B) TYPE: amino acid
(C) TOPOLOGY: linear


(ii) SEQUENCE DESCRIPTION: SEQ ID NO: 6


Gly Glu Gly Phe Leu Gly Asp Phe Leu
1                    5



(8) INFORMATION FOR SEQ ID NO: 7


(i) SEQUENCE CHARACTERISTICS:


(A) LENGTH: 11 amino acids
(B) TYPE: amino acid
(C) TOPOLOGY: linear


(ii) SEQUENCE DESCRIPTION: SEQ ID NO: 7


Ile Ser Glu Val Lys Met Asp Ala Glu Phe
1                    5                    10


Arg

(9)   INFORMATION FOR SEQUENCE ID NO: 8

    (i)   SEQUENCE CHARACTERISTICS:

        (A)   LENGTH:   39 nucleotides
        (B)   TYPE:   nucleic acids
        (C)   STRANDEDNESS:   single strand
        (D)   TOPOLOGY:   linear

    (ii)   MOLECULAR TYPE:

        cDNA to mRNA

    (iii) PUBLICATION INFORMATION:

        (A)   AUTHORS:   Kang et al.
        (B)   JOURNAL:   Nature
        (C)   VOLUME:   325
        (D)   PAGE:   733
        (E)   DATE:   1987

    (iv)   SEQUENCE DESCRIPTION: SEQ ID NO: 8

        CTCTAGAACT AGTGGGTCGA CACGATGCTG CCCGGTTTG     39